# EUROPEAN PATENT SPECIFICATION

(11) **EP 3 756 917 B1**
(45) Date of publication and mention of the grant of the patent: **19.06.2024**
(21) Application number: 20181827.5
(22) Date of filing: 24.06.2020
(51) Int. Cl.: B60H 1/00, B60H 3/00, B60H 3/02, A61L 9/00, A61L 9/12, A61L 9/22

(54) **VEHICULAR AIR CONDITIONER**
FAHRZEUGKLIMAANLAGE
CLIMATISEUR DE VÉHICULE

(30) Priority: 28.06.2019 JP 2019120764
(43) Date of publication of application: 30.12.2020
(73) Proprietor: Valeo Japan Co., Ltd., Kumagaya-shi, Saitama 360-0193 (JP)
(72) Inventor: SAKURAI, Yusaku, Saitama 360-0193 (JP); NAGANO, Hideki, Saitama 360-0193 (JP); TANAKA, Yuya, Saitama 360-0193 (JP); NAKAMURA, Saki, Saitama 360-0193 (JP)
(74) Representative: Valeo Systèmes Thermiques

(56) References cited:
- DE-A1-102008 049 278
- FR-A1- 3 011 743
- JP-A- 2006 010 220
- JP-A- 2008 189 246
- US-A1- 2018 370 334

## Description

### BACKGROUND OF THE INVENTION

### 1. Field of the Invention

The present invention relates to vehicular air conditioners capable of sufficiently supplying, into a cabin, air to which a function, such as ions and fragrance, has been added and, in particular, relates to a vehicular air conditioner including an air conditioning case divided into a plurality of passages individually controllable in temperature.

### 2. Description of the Related Art

For example, document JP-A-2008-254549 discloses an air conditioner for a vehicle to relieve or remove electrostatic charge from an occupant at the time of getting off the vehicle. For this purpose, an ion generator is disposed in a blowing path of conditioned air, and such a setting is made that a blowout opening of the air conditioner when the occupant's intention to get off the vehicle is detected is a blowout opening through which the air is blown to the occupant, and that while a fan is blowing the air, the ion generator generates ions in the blowing path.

Particularly in this example, the ion generator to supply ions to the conditioned air is disposed in the blowing path between a heater core and a vent duct of the air conditioner, more specifically, downstream of an evaporator of an air conditioning case of the air conditioner and upstream of a portion where a defrost duct is coupled. When the occupant's intention to get off the vehicle is detected, the flow of air is switched to blowout from a vent outlet, and the ion generator is operated to add ions to air that has been humidified by condensed water attached to the evaporator, and to supply the ion-added air into the cabin via the vent outlet.

However, it matters how the ion generator is installed in the vehicular air conditioner including the air conditioning case divided into a plurality of passages by a partition wall to supply differently conditioned air to different areas in the cabin.

For example, document JP-A-09-249017 discloses an air conditioner capable of left-and-right individual temperature control to individually control temperatures of air flows to a driver's seat side and a front passenger's seat side of a cabin. In this air conditioner, an air passage in an air conditioning case is divided into left and right passages (divisional passages) by a partition plate, and opening degrees of air mixing doors and switching doors of blowout openings in each of the divisional passages are adjusted to supply differently temperature-controlled air to the driver's seat area and the front passenger' s seat area (see Patent Literature 2). The term "front passenger's seat" will be hereinafter simply referred to as "passenger's seat".

In such an air conditioner, when an ion generator is disposed in one of the divisional passages, ions are only added to air blown out of this divisional passage. This results in inconvenience that ions cannot be added to air blown out of the other divisional passage.

Document DE 10 2008 049278 A1 discloses also a similar vehicular air conditioner comprising a function device adding ions in the separated air passages.

In the vehicular air conditioner having the air conditioning case divided into the plurality of passages individually controllable in temperature, there is need to provide an ion generator for each of the divisional passages to supply ion-added air to any area in the cabin. Inconveniently, this not only increases the costs but also makes it difficult to install a plurality of ion generators in a limited installation space in the air conditioner.

In view of this, it can be considered not to dispose the ion generator downstream of a heat exchanger where the partition plate is provided but to dispose the ion generator upstream of the heat exchanger upstream of the partition plate. However, with the ion generator being disposed upstream of the heat exchanger, ions generated by the ion generator come into contact with the heat exchanger and other components and disappear, which results in inconvenience that an amount of the ions that can be supplied to the cabin becomes insufficient.

Such an inconvenient situation may similarly occur in providing the air conditioner with a function adding device to add other functions such as fragrance as well as in installing the ion generator to add ions to air.

### SUMMARY OF THE INVENTION

The invention has been achieved in consideration of the above-described circumstances. An object of the invention is mainly to provide a vehicular air conditioner in which an air conditioning case includes a plurality of divisional passages divided by a partition wall and individually controllable in temperature so that even when a single function adding device is installed, air to which a function has been added can be sufficiently supplied to a cabin from any of the divisional passages.

In order to attain the object, a vehicular air conditioner configured to blow air to which a function has been added into a cabin according to claim 1 is provided. Such air conditioner includes an air conditioning case and a heat exchanger. The air conditioning case includes an internal air passage and a plurality of blowout openings capable of supplying intake air into the cabin. The heat exchanger is disposed in the air conditioning case and configured to perform heat exchange of the intake air. The air passage at least downstream of the heat exchanger is divided into a plurality of divisional passages by a partition wall. A space defining wall member is disposed on a wall portion of the air conditioning case. The wall portion is configured to change a flowing direction of the air that has passed through the heat exchanger. The space defining wall member is configured to define a function addition space between the space defining wall member and the wall portion. The function addition space is not divided by the partition wall. A function adding device is disposed on the wall portion or the space defining wall member and configured to add a predetermined function to the air flowing through the function addition space. The function addition space is configured to communicate with each of the divisional passages via at least two communicating portions. At least one of the communicating portions in each of the divisional passages is configured to be opened toward one of the blowout openings so that a pressure in the at least one of the communicating portions is lower than a pressure in another of the communicating portions in a predetermined blowout mode.

In this air conditioner, the communicating portions may be openings formed in the space defining wall member and the wall portion or may be intervals between the space defining wall member and the wall portion.

Thus, in the vehicular air conditioner in which the air passage at least downstream of the heat exchanger is divided into the plurality of divisional passages by the partition wall, the space defining wall member is disposed on the wall portion of the air conditioning case to change the flowing direction of the air that has passed through the heat exchanger. The space defining wall member defines the function addition space between the space defining wall member and the wall portion and is not divided by the partition wall. The function adding device is disposed on the wall portion or the space defining wall member and adds the predetermined function to the air flowing through the function addition space. The function addition space communicates with each of the divisional passages via the at least two communicating portions. The at least one of the communicating portions in each of the divisional passages is opened toward one of the blowout openings so that the pressure in the at least one of the communicating portions is lower than the pressure in the other of the communicating portions in the predetermined blowout mode . Consequently, the function addition space can be shared by the divisional passages. In each of the divisional passages, in the predetermined blowout mode, the air is taken into the function addition space by suction from the other of the communicating portions, and after the function is added to the suction air in the function addition space by the function adding device, the air is returned to the divisional passage from the communicating portion having a relatively low pressure (at least one of the communicating portions in each of the divisional passages can be made to have an effect of drawing the air that has passed through the function addition space) . Therefore, no matter from which of the divisional passages the conditioned air is supplied to the cabin, the air to which the function has been added in the function addition space can be supplied. This makes it possible to commonly use the function adding device to add the function in the function addition space so that there is no need to provide the function adding device for each of the divisional passages.

According to the invention, the plurality of blowout openings includes a vent blowout opening configured to supply air blown toward an upper portion of the cabin, and a foot blowout opening configured to supply air blown toward a lower portion of the cabin. In particular, to sufficiently supply the function-added air from the vent blowout opening, the space defining wall member is disposed on the wall portion between the vent blowout opening and the foot blowout opening, and the partition wall is configured to divide the air passage at least downstream of the heat exchanger into a driver's seat-side divisional passage and a passenger's seat-side divisional passage, divide the vent blowout opening into a driver's seat-side vent blowout opening and a passenger' s seat-side vent blowout opening, and divide the foot blowout opening into a driver's seat-side foot blowout opening and a passenger's seat-side foot blowout opening.

The function addition space is configured to communicate with the driver's seat-side divisional passage via a first driver's seat-side communicating portion disposed close to the driver's seat-side vent blowout opening and a second driver's seat-side communicating portion disposed close to the driver's seat-side foot blowout opening, and the function addition space is configured to communicate with the passenger's seat-side divisional passage via a first passenger's seat-side communicating portion disposed close to the passenger's seat-side vent blowout opening and a second passenger's seat-side communicating portion disposed close to the passenger's seat-side foot blowout opening.

Preferentially, in a blowout mode in which the driver's seat-side vent blowout opening is open, the first driver's seat-side communicating portion may be configured to be opened toward the driver's seat-side vent blowout opening to make the first driver's seat-side communicating portion have a lower pressure than the second driver's seat-side communicating portion, and in a blowout mode in which the passenger's seat-side vent blowout opening is open, the first passenger's seat-side communicating portion may be configured to be opened toward the passenger's seat-side vent blowout opening to make the first passenger's seat-side communicating portion have a lower pressure than the second passenger's seat-side communicating portion.

In the above-described configuration, the space defining wall member is disposed on the wall portion between the vent blowout opening and the foot blowout opening. In the driver's seat-side divisional passage, the first driver's seat-side communicating portion is disposed close to the driver's seat-side vent blowout opening. In the blowout mode (a vent blowout mode or a bi-level blowout mode) in which the driver's seat-side vent blowout opening is open, the first driver's seat-side communicating portion is configured to be opened toward the driver's seat-side vent blowout opening to make the first driver's seat-side communicating portion have a lower pressure than the second driver's seat-side communicating portion disposed close to the driver's seat-side foot blowout opening. In the passenger's seat-side divisional passage, the first passenger's seat-side communicating portion is disposed close to the passenger's seat-side vent blowout opening. In the blowout mode (the vent blowout mode or the bi-level blowout mode) in which the passenger's seat-side vent blowout opening is open, the first passenger' s seat-side communicating portion is configured to be opened toward the passenger's seat-side vent blowout opening to make the first passenger's seat-side communicating portion have a lower pressure than the second passenger's seat-side communicating portion disposed close to the passenger's seat-side foot blowout opening. Consequently, when the blowout mode (the vent blowout mode or the bi-level blowout mode) in which the vent blowout opening is open is set on one or both of the driver's seat side and the passenger's seat side, the air is taken into the function addition space by suction via the second communicating portion(s) from the divisional passage(s). After the function is added to the suction air in the function addition space by the function adding device, the air is returned to the divisional passage (s) (the driver's seat-side divisional passage and/or the passenger's seat-side divisional passage) set in the blowout mode via the first communicating portion (s) (the first driver' s seat-side communicating portion and/or the first passenger's seat-side communicating portion) (the first communicating portion (s) have an effect of drawing the air from the function addition space). Therefore, the air to which the function has been added in the function addition space can be effectively introduced into the vent blowout opening in the divisional passage set in the blowout mode.

Preferentially, in a blowout mode in which the driver's seat-side foot blowout opening is open, the second driver's seat-side communicating portion may be configured to be opened toward the driver's seat-side foot blowout opening to make the second driver's seat-side communicating portion have a lower pressure than the first driver's seat-side communicating portion, and in a blowout mode in which the passenger's seat-side foot blowout opening is open, the second passenger's seat-side communicating portion may be configured to be opened toward the passenger's seat-side foot blowout opening to make the second passenger's seat-side communicating portion have a lower pressure than the first passenger's seat-side communicating portion.

In the above-described configuration, the space defining wall member is disposed on the wall portion between the vent blowout opening and the foot blowout opening. In the driver's seat-side divisional passage, the second driver's seat-side communicating portion is disposed close to the driver's seat-side foot blowout opening. In the blowout mode (a foot blowout mode) in which the driver's seat-side foot blowout opening is open, the second driver's seat-side communicating portion is configured to be opened toward the driver's seat-side foot blowout opening to make the second driver's seat-side communicating portion have a lower pressure than the first driver's seat-side communicating portion disposed close to the driver's seat-side vent blowout opening. In the passenger's seat-side divisional passage, the second passenger's seat-side communicating portion is disposed close to the passenger's seat-side foot blowout opening. In the blowout mode (the foot blowout mode) in which the passenger's seat-side foot blowout opening is open, the second passenger's seat-side communicating portion is configured to be opened toward the passenger's seat-side foot blowout opening to make the second passenger's seat-side communicating portion have a lower pressure than the first passenger's seat-side communicating portion disposed close to the passenger's seat-side vent blowout opening. Consequently, when the blowout mode (the foot blowout mode) in which the foot blowout opening is open is set on one or both of the driver's seat side and the passenger's seat side, the air is taken into the function addition space by suction via the first communicating portion (s) from the divisional passage (s) . After the function is added to the suction air in the function addition space by the function adding device, the air is returned to the divisional passage (s) (the driver's seat-side divisional passage and/or the passenger's seat-side divisional passage) set in the blowout mode via the second communicating portion (s) (the second driver's seat-side communicating portion and/or the second passenger's seat-side communicating portion) (the second communicating portion (s) have an effect of drawing the air from the function addition space) . Therefore, the air to which the function has been added in the function addition space can be effectively introduced into the foot blowout opening in the divisional passage set in the blowout mode.

In this air conditioner, in each of the divisional passages, the communicating portion relatively low in pressure may be formed closer to the blowout opening than the other of the communicating portions is to the blowout opening.

In this manner, the space defining wall member includes the communicating portion relatively low in pressure that is formed closer to the blowout opening than the other of the communicating portions is so that the effect of drawing the air from the function addition space can be enhanced.

In each of the divisional passages, the space defining wall member may be disposed inside of the wall portion (disposed inside of the air conditioning case) , and a surface of the space defining wall member against which the air flowing from an upstream side collides may have an intermediate portion protruding toward the upstream side.

In this configuration, the intermediate portion of the surface (upstream wall surface) of the space defining wall member against which the air flowing from the upstream side collides protrudes toward the upstream side so that the air flowing from the upstream side can be smoothly divided at the upstream wall surface of the space defining wall member and made to flow toward the communicating portion. This makes it easier to obtain the effect of drawing the air from the function addition space.

In the divisional passages, a guide portion may be disposed on or in a vicinity of a portion of the space defining wall member where the other of the communicating portions is formed, and the guide portion is configured to promote introduction of the air into the other of the communicating portions.

As such a guide portion, the space defining wall member may be expanded toward a mixing area to increase a passage cross-sectional area of the other of the communicating portions toward an open end, and a guide wall to block air flowing away from the function addition space may be disposed in the vicinity of the other of the communicating portions.

The function addition space defined by the space defining wall member may include an intermediate portion having a passage cross-sectional area smaller than the communicating portions.

In this configuration, the air taken into the function addition space by suction receives the function from the function adding device, is increased in flowrate at the intermediate portion, and is thereafter discharged from the communicating portion having a relatively large passage cross-sectional area. Thus, without colliding against the space defining wall member, the function-added air can be quickly supplied to the blowout openings. This makes it possible to avoid inconvenience of the function-added air being decreased in function by colliding against the space defining wall member.

The space defining wall member may be disposed outside of the wall portion of the air conditioning case. With this configuration, the space defining wall member can be attached outside the air conditioning case afterward to facilitate manufacturing the air conditioner.

In the above-described vehicular air conditioner, when the heat exchanger includes a cooling heat exchanger configured to cool the air passing therethrough and a heating heat exchanger disposed downstream of the cooling heat exchanger and configured to heat the air passing therethrough, the heating heat exchanger may be arranged in the air passage in such a manner that bypasses are formed above and below the heating heat exchanger or may be arranged in the air passage in such a manner that bypasses are formed only above the heating heat exchanger.

The function added to the air by the function adding device may include at least one of an ion adding function, a fragrance function, a deodorizing function, a humidity control function, and a sterilizing function.

As described above, in the vehicular air conditioner according to the invention, the function addition space can be shared by the divisional passages, and even when the single function adding device is provided, the air to which the function has been added can be sufficiently supplied to the cabin via any of the divisional passages.

### BRIEF DESCRIPTION OF THE DRAWINGS

Fig. 1A is a side cross-sectional view of a vehicular air conditioner according to an embodiment of the invention, illustrating an exemplary configuration of a downstream side of a heat exchanger.
Fig. 1B is a plan view of a portion of the air conditioner where blowout openings are formed.
Fig. 2 is a plan cross-sectional view of the vehicular air conditioner according to the embodiment of the invention, illustrating an exemplary configuration of the downstream side of the heat exchanger, taken along line A-A in Fig. 1A.
Figs. 3A and 3B are respectively a side cross-sectional view and a perspective view of a space defining wall member and an ion generator, illustrating an attachment state thereof.
Fig. 4 is a diagram illustrating air flows in a vent blowout mode.
Fig. 5 is a diagram illustrating air flows in a bi-level blowout mode.
Figs. 6A to 6C illustrate a mode (an all seats mode) in which air to which ions have been added is supplied to a driver's seat side and a passenger's seat side, of which Fig. 6A is a front view illustrating air flows in a function addition space, Fig. 6B is a side cross-sectional view illustrating air flows in a driver's seat-side divisional passage, and Fig. 6C is a side cross-sectional view illustrating air flows in a passenger's seat-side divisional passage.
Figs. 7A to 7C illustrate a mode (a driver's seat prior mode) in which air to which ions have been added is supplied only to the driver's seat side, of which Fig. 7A is a front view illustrating air flows in the function addition space, Fig. 7B is a side cross-sectional view illustrating air flows in the driver's seat-side divisional passage, and Fig. 7C is a side cross-sectional view illustrating air flows in the passenger's seat-side divisional passage.
Figs. 8A to 8C illustrate a mode (a passenger's seat prior mode) in which air to which ions have been added is supplied only to the passenger's seat side, of which Fig. 8A is a front view illustrating air flows in the function addition space, Fig. 8B is a side cross-sectional view illustrating air flows in the driver's seat-side divisional passage, and Fig. 8C is a side cross-sectional view illustrating air flows in the passenger's seat-side divisional passage.
Fig. 9 is a diagram illustrating air flows in a foot blowout mode.
Fig. 10 is a diagram illustrating an example in which the space defining wall member is extended toward vent blowout openings.
Fig. 11 is a diagram illustrating an example in which a portion of the space defining wall member that is close to the vent blowout openings is bent along air flows at the vent blowout openings.
Fig. 12 is a diagram illustrating an example in which an intermediate portion of the space defining wall member is protruded upstream.
Fig. 13 is a diagram illustrating an example in which a guide wall is disposed immediately under the space defining wall member.
Fig. 14 is a diagram illustrating an example in which an open end portion of the space defining wall member that is close to foot blowout openings is expanded upstream to increase a passage area of the open end portion.
Figs. 15A and 15B illustrate another example of the space defining wall member, of which Fig. 15A is a perspective view of the space defining wall member as viewed from a front upper side, and Fig. 15B is a perspective view of the space defining wall member as viewed from a diagonal upper side.
Fig. 16 is a diagram illustrating an example of the air conditioner in which bypasses are formed only above a heater core.
Figs. 17A and 17B are respectively a side cross-sectional view and a perspective view, illustrating an example in which the space defining wall member is disposed outside of a wall portion of an air conditioning case.
Figs. 18A and 18B illustrate an exemplary configuration of supplying ion-added air to the foot blowout openings, of which Fig. 18A is a diagram illustrating the case of a defrost-foot blowout mode, and Fig. 18B is a diagram illustrating the case of the foot blowout mode.

### DESCRIPTION OF THE PREFERRED EMBODIMENTS

An embodiment of the invention will now be described with reference to the accompanying drawings.

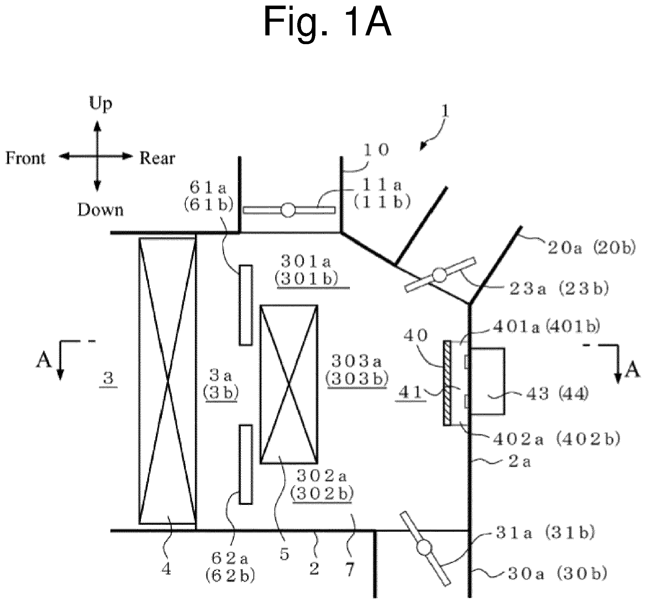

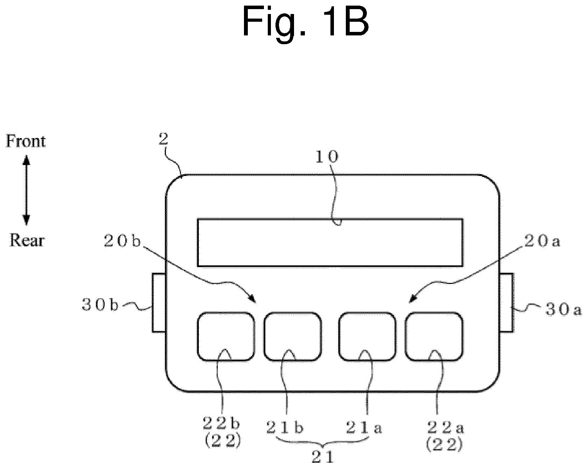

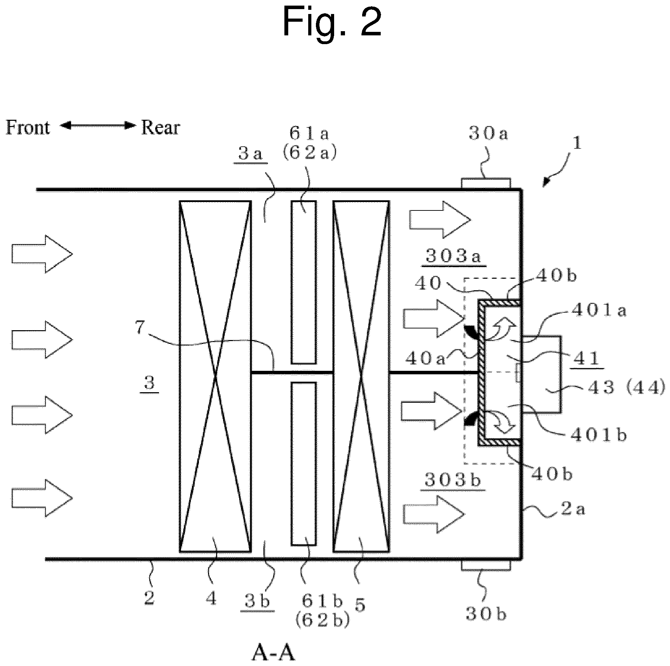

Figs. 1A, 1B, and 2 illustrate a centered-type vehicular air conditioner 1 (of a fully-centered type and a semi-centered type) mounted substantially in the center of a dashboard of a vehicle. This air conditioner 1 is disposed closer to a cabin than to a partition plate that divides an engine room and the cabin. The air conditioner 1 introduces outside air (air outside of the cabin) and/or inside air (air inside of the cabin) into an air conditioning case 2 through an intake device, not illustrated.

The air conditioning case 2 forms an air passage 3 where the air flows toward the cabin. An evaporator 4 is a cooling heat exchanger capable of cooling the intake air and disposed at an upstream side of the air passage 3. A heater core 5 is a heating heat exchanger and disposed downstream of the evaporator 4.

The evaporator 4 constitutes part of a freezing cycle, stands in the air passage 3 to allow all the air that has been introduced into the air conditioning case 2 to pass through the evaporator 4, and is capable of cooling the air passing therethrough as necessary.

The heater core 5 heats the air passing therethrough using coolant of the engine as a heat source. In this embodiment, the heater core 5 stands in such a manner that intervals are formed between the heater core 5 and an upper surface and a lower surface of the air conditioning case 2.

In this embodiment, a portion of the air passage 3 downstream of the evaporator 4 is divided into two left and right divisional passages (a driver's seat-side divisional passage 3a and a passenger's seat-side divisional passage 3b) by a partition wall 7. The evaporator 4 and the heater core 5 are disposed over both sides of the partition wall 7 and shared by the divisional passages 3a, 3b.

Consequently, in the respective divisional passages 3a, 3b, upper bypasses (a driver's seat-side upper bypass 301a and a passenger's seat-side upper bypass 301b) are formed above the heater core 5 in such a manner that the air that has passed through the evaporator 4 does not pass through the heater core 5 but is bypassed, and lower bypasses (a driver's seat-side lower bypass 302a and a passenger's seat-side lower bypass 302b) are formed below the heater core 5 in such a manner that the air that has passed through the evaporator 4 does not pass through the heater core 5 but is bypassed.

It should be noted that the bypasses 301a, 301b, 302a, 302b may be formed by adjusting an installation height of the heater core 5 in the air conditioning case 2 or by recessing the upper surface and the lower surface of the air conditioning case 2 that are opposed to the heater core 5 in directions away from the heater core 5.

In the respective divisional passages 3a, 3b, ratios of the air flowing through the bypasses 301a, 301b, 302a, 302b to the air passing through the heater core 5 are adjustable by air mixing doors 61a, 61b, 62a, 62b disposed upstream of the heater core 5.

In this embodiment, in the driver's seat-side divisional passage 3a, an opening degree of the driver's seat-side upper bypass 301a is adjusted by the driver's seat-side upper air mixing door 61a slidable along an end surface of the heater core 5, and an opening degree of the driver's seat-side lower bypass 302a is adjusted by the driver's seat-side lower air mixing door 62a slidable along the end surface of the heater core 5. In the passenger's seat-side divisional passage 3b, an opening degree of the passenger's seat-side upper bypass 301b is adjusted by the passenger's seat-side upper air mixing door 61b slidable along the end surface of the heater core 5, and an opening degree of the passenger' s seat-side lower bypass 302b is adjusted by the passenger's seat-side lower air mixing door 62b slidable along the end surface of the heater core 5.

Thus, the evaporator 4, the heater core 5, the driver's seat-side upper air mixing door 61a, and the driver's seat-side lower air mixing door 62a constitute a driver's seat-side temperature controller to control a temperature of the air introduced into the driver's seat-side divisional passage 3a. Also, the evaporator 4, the heater core 5, the passenger's seat-side upper air mixing door 61b, and the passenger's seat-side lower air mixing door 62b constitute a passenger's seat-side temperature controller to control a temperature of the air introduced into the passenger's seat-side divisional passage 3b. This makes the air flowing through each of the divisional passages 3a, 3b individually controllable in temperature.

The heater core 5, the bypasses (the driver's seat-side upper bypass 301a and the driver's seat-side lower bypass 302a) , and a driver's seat-side mixing area 303a capable of mixing the air that has passed through the heater core 5 are formed downstream of the evaporator 4 in the driver's seat-side divisional passage 3a. In a portion of the air conditioning case 2 that faces the driver's seat-side mixing area 303a, a plurality of driver's seat-side blowout openings are formed to supply air to be blown out toward space at the driver's seat in the cabin.

The heater core 5, the bypasses (the passenger's seat-side upper bypass 301b and the passenger' s seat-side lower bypass 302b), and a passenger's seat-side mixing area 303b capable of mixing the air that has passed through the heater core 5 are formed downstream of the evaporator 4 in the passenger's seat-side divisional passage 3b. In a portion of the air conditioning case 2 that faces the passenger's seat-side mixing area 303b, a plurality of passenger's seat-side blowout openings are formed to supply air to be blown out toward space at the passenger's seat in the cabin.

In an upper front portion of the air conditioning case 2 that faces the driver's seat-side mixing area 303a and in an upper front portion of the air conditioning case 2 that faces the passenger's seat-side mixing area 303b, a common defrost blowout opening 10 is formed to supply air to be blown out toward the windshield of the vehicle.

In an upper rear portion of the air conditioning case 2 that faces the driver's seat-side mixing area 303a, a driver's seat-side vent blowout opening 20a is formed to supply air to be blown out toward an upper side of the driver's seat. In an upper rear portion of the air conditioning case 2 that faces the passenger's seat-side mixing area 303b, a passenger's seat-side vent blowout opening 20b is formed to supply air to be blown out toward an upper side of the passenger's seat.

In this embodiment, the vent blowout openings (the driver's seat-side vent blowout opening 20a and the passenger' s seat-side vent blowout opening 20b) include center vent blowout openings 21 (a driver's seat-side center vent blowout opening 21a and a passenger's seat-side center vent blowout opening 21b) disposed close to the center in a lateral direction (a vehicle width direction) and side vent blowout openings 22 (a driver's seat-side side vent blowout opening 22a and a passenger's seat-side side vent blowout opening 22b) adjacent to the center vent blowout openings 21 and disposed sideward in the lateral direction (the vehicle width direction).

In a lower portion of the air conditioning case 2 that faces the driver's seat-side mixing area 303a, a driver's seat-side foot blowout opening 30a is formed to supply air to be blown out toward a lower side of the driver's seat. In a lower portion of the air conditioning case 2 that faces the passenger's seat-side mixing area 303b, a passenger's seat-side foot blowout opening 30b is formed to supply air to be blown out toward a lower side of the passenger's seat. In this embodiment, the driver's seat-side foot blowout opening 30a is formed in a lower portion of a driver's seat-side side surface of the air conditioning case 2, and the passenger's seat-side foot blowout opening 30b is formed in a lower portion of a passenger, s seat-side side surface of the air conditioning case 2.

Hence, on the cabin side of the air conditioning case 2, a wall portion 2a is formed in a region between the pair of the driver's seat-side vent blowout opening 20a and the passenger's seat-side vent blowout opening 20b and the pair of the driver's seat-side foot blowout opening 30a and the passenger's seat-side foot blowout opening 30b. The air that has passed through the heat exchangers (the evaporator 4 and the heater core 5) in each of the divisional passages 3a, 3b collides against the wall portion 2a and changes in flowing direction.

It should be noted that an opening degree of the defrost blowout opening 10 is adjusted by a driver's seat-side defrost door 11a and a passenger's seat-side defrost door 11b that are disposed inside of the defrost blowout opening 10. An opening degree of the driver's seat-side vent blowout opening 20a (the driver's seat-side center vent blowout opening 21a and the driver's seat-side side vent blowout opening 22a) is adjusted by a driver's seat-side vent door 23a that faces the driver's seat-side vent blowout opening 20a (the driver's seat-side center vent blowout opening 21a and the driver's seat-side side vent blowout opening 22a). An opening degree of the passenger's seat-side vent blowout opening 20b (the passenger's seat-side center vent blowout opening 21b and the passenger's seat-side side vent blowout opening 22b) is adjusted by a passenger's seat-side vent door 23b that faces the passenger's seat-side vent blowout opening 20b (the passenger's seat-side center vent blowout opening 21b and the passenger's seat-side side vent blowout opening 22b).

In this embodiment, the opening degrees of the driver's seat-side side vent blowout opening 22a and the passenger's seat-side side vent blowout opening 22b are adjusted to secure constant flowrates irrespective of blowout modes.

An opening degree of the driver's seat-side foot blowout opening 30a is adjusted by a driver's seat-side foot door 31a that faces the driver's seat-side foot blowout opening 30a. An opening degree of the passenger's seat-side foot blowout opening 30b is adjusted by a passenger's seat-side foot door 31b that faces the passenger's seat-side foot blowout opening 30b.

In such an air conditioner 1, a space defining wall member 40 is disposed on the wall portion 2a of the air conditioning case 2 to change the flowing direction of the air that has passed through the heat exchangers (the evaporator 4 and the heater core 5).

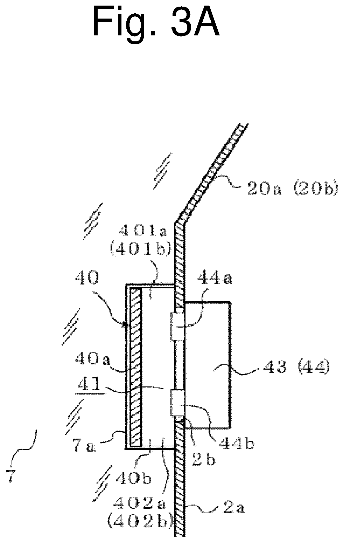

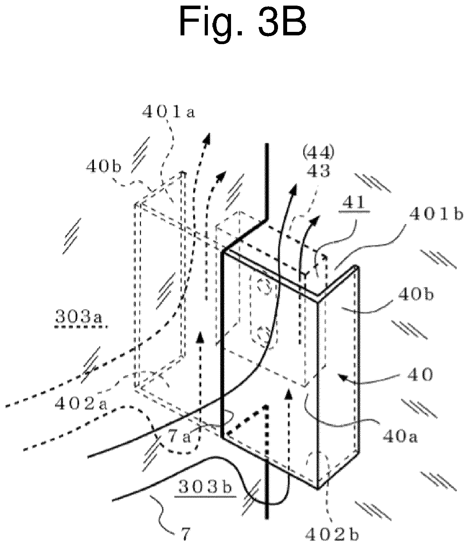

As illustrated in Figs. 3A and 3B, the space defining wall member 40 is attached to a notch 7a in the partition wall 7 and protrudes into both of the driver's seat-side divisional passage 3a and the passenger's seat-side divisional passage 3b. The space defining wall member 40 includes a rectangular front wall 40a and side walls 40b. The front wall 40a is opposed to and spaced from the wall portion 2a of the air conditioning case 2 with a predetermined interval while crossing the partition wall 7. The side walls 40b each extend to the wall portion 2a of the air conditioning case 2 from a side edge of the front wall 40a that protrudes into each of the divisional passages 3a, 3b. The space defining wall member 40 is of a shape having an upper end portion and a lower end portion opened.

Consequently, between the wall portion 2a of the air conditioning case 2 and the space defining wall member 40, a space (a function addition space 41) covered with the space defining wall member 40 and not divided by the partition wall 7 is defined and constantly communicates with the driver's seat-side divisional passage 3a and the passenger's seat-side divisional passage 3b.

Specifically, the function addition space 41 communicates with the driver's seat-side divisional passage 3a (the driver's seat-side mixing area 303a) via a first driver's seat-side communicating portion 401a that is formed in the interval between the space defining wall member 40 and the wall portion 2a and close to the driver's seat-side vent blowout opening 20a and that is opened toward the driver's seat-side vent blowout opening 20a. The function addition space 41 also communicates with the driver's seat-side divisional passage 3a (the driver's seat-side mixing area 303a) via a second driver's seat-side communicating portion 402a that is formed in the interval between the space defining wall member 40 and the wall portion 2a and close to the driver's seat-side foot blowout opening 30a.

The function addition space 41 communicates with the passenger's seat-side divisional passage 3b (the passenger's seat-side mixing area 303b) via a first passenger's seat-side communicating portion 401b that is formed in the interval between the space defining wall member 40 and the wall portion 2a and close to the passenger's seat-side vent blowout opening 20b and that is opened toward the passenger's seat-side vent blowout opening 20b. The function addition space 41 also communicates with the passenger's seat-side divisional passage 3b (the passenger's seat-side mixing area 303b) via a second passenger's seat-side communicating portion 402b that is formed in the interval between the space defining wall member 40 and the wall portion 2a and close to the passenger's seat-side foot blowout opening 30b.

It should be noted that in this embodiment, the function addition space 41 has a uniform cross-sectional area from an upper end to a lower end.

A function adding device 43 to add a predetermined function to air flowing through the function addition space 41 is disposed on the wall portion 2a of the air conditioning case 2 covered with the space defining wall member 40.

In this embodiment, the function adding device 43 serves as an ion generator 44 to add ions to the air flowing through the function addition space 41. The ion generator 44 is attached outside of the wall portion 2a of the air conditioning case 2 in such a manner that a cathode 44a and an anode 44b to generate ions face the function addition space 41 through a through hole 2b formed in the wall portion 2a.

In this embodiment, the first communicating portion (the first driver's seat-side communicating portion 401a and the first passenger's seat-side communicating portion 401b) and the second communicating portion (the second driver's seat-side communicating portion 402a and the second passenger's seat-side communicating portion 402b) are vertically symmetrical with respect to the ion generator 44. On the wall portion 2a on the cabin side, the space defining wall member 40 is closer to the vent blowout openings 20 than to the foot blowout openings 30. The first communicating portions 401a, 401b are opened toward the vent blowout openings 20a, 20b so that in a blowout mode in which the vent blowout openings 20a, 20b are open, an air flow around the space defining wall member 40 makes static pressure in the first communicating portions 401a, 401b lower than static pressure in the second communicating portions 402a, 402b.

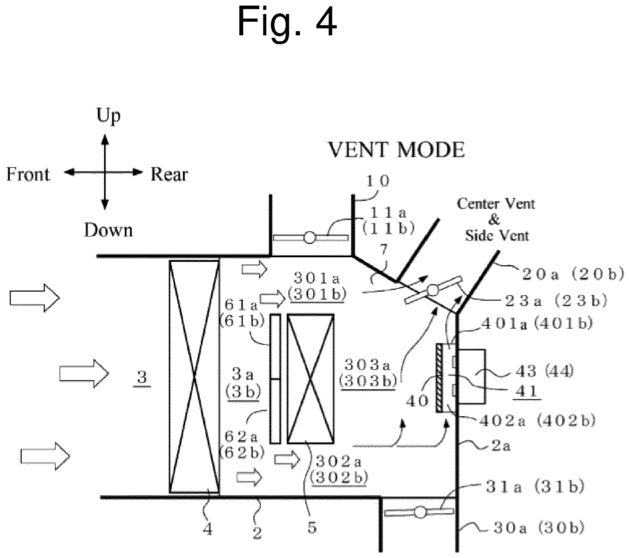

With the above-described configuration, the case of setting a vent blowout mode will now be described. In the vent blowout mode, as illustrated in Fig. 4, for example, in each of the driver's seat-side divisional passage 3a and the passenger's seat-side divisional passage 3b, the air mixing doors 61a, 61b, 62a, 62b occupy "full cool" positions (positions to cause all of the air that has passed through the evaporator 4 to bypass the heater core 5 and flow through the bypasses 301a, 301b, 302a, 302b), and only the vent blowout opening 20a, 20b is opened.

In such a blowout mode, in each of the divisional passages (the driver's seat-side divisional passage 3a, the passenger's seat-side divisional passage 3b), the air that has been supplied from an upstream side of the heat exchangers 4 and 5 in the air conditioning case 2 passes through the evaporator 4, flows through the upper bypass (the driver's seat-side upper bypass 301a, the passenger's seat-side upper bypass 301b) and the lower bypass (the driver's seat-side lower bypass 302a, the passenger's seat-side lower bypass 302b), and reaches the mixing area (the driver's seat-side mixing area 303a, the passenger's seat-side mixing area 303b).

The air that has been guided into the mixing area 303a, 303b collides against the front wall 40a of the space defining wall member 40 and the wall portion 2a of the air conditioning case 2 and is changed to flow upward and guided along a surface of the space defining wall member 40 and the wall portion 2a of the air conditioning case 2 to the vent blowout opening 20a, 20b. Meanwhile, the air that has been guided into the mixing area 303a, 303b enters the function addition space 41 from each of the divisional passages (the driver's seat-side divisional passage 3a, the passenger's seat-side divisional passage 3b) via the second communicating portion (the second driver's seat-side communicating portion 402a, the second passenger's seat-side communicating portion 402b). After ions generated by the ion generator 44 are added to the air, the air is returned to the air passage 3 (the mixing area 303a, 303b) via the first communicating portion (the first driver's seat-side communicating portion 401a, the first passenger's seat-side communicating portion 401b) and guided to the vent blowout opening (the driver's seat-side vent blowout opening 20a, the passenger's seat-side vent blowout opening 20b).

At this time, in the respective divisional passages 3a, 3b, the air flowing along the surface of the space defining wall member 40 to the vent blowout openings (the driver's seat-side vent blowout opening 20a and the passenger's seat-side vent blowout opening 20b) causes an aspiration effect in the first communicating portions (the first driver's seat-side communicating portion 401a and the first passenger's seat-side communicating portion 401b), thereby making the static pressure in the first driver's seat-side communicating portion 401a and the first passenger's seat-side communicating portion 401b lower than the static pressure in the second driver's seat-side communicating portion 402a and the second passenger's seat-side communicating portion 402b. Consequently, the air in the function addition space 41 is drawn in (taken in by suction) to the driver's seat-side vent blowout opening 20a via the first driver's seat-side communicating portion 401a and drawn in (taken in by suction) to the passenger's seat-side vent blowout opening 20b via the first passenger's seat-side communicating portion 401b. Also, the air in the driver's seat-side mixing area 303a is taken in by suction to the function addition space 41 via the second driver's seat-side communicating portion 402a, and the air in the passenger's seat-side mixing area 303b is taken in by suction to the function addition space 41 via the second passenger's seat-side communicating portion 402b.

Thus, the air to which ions have been added in the function addition space 41 is supplied to each of the divisional passages 3a, 3b so that use of the single ion generator 44 enables supply of the ion-added air to the cabin from each of the divisional passages 3a, 3b. This eliminates need to provide each of the divisional passages with an ion generator and prevents inconvenient trouble even when the air conditioner is installed in a limited space.

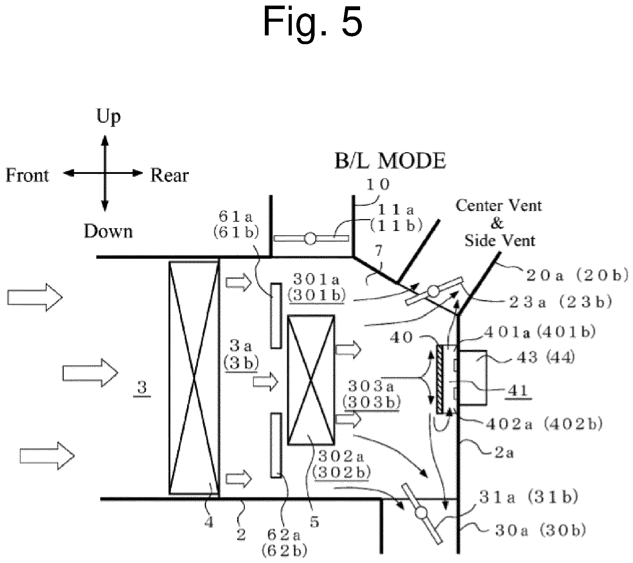

Next, the case of setting a bi-level blowout mode will be described. In the bi-level blowout mode, as illustrated in Fig. 5, for example, on the driver's seat-side and the passenger's seat-side, respectively, the air mixing doors 6a, 6b occupy intermediate positions (positions to cause the air that has passed through the evaporator 4 to pass through the heater core 5 and flow through the bypasses (the upper bypasses 301a, 301b and the lower bypasses 302a, 302b)), and the vent blowout openings 20a, 20b and the foot blowout openings 30a, 30b are open.

In this blowout mode, the air supplied to the air conditioning case 2 passes through the evaporator 4 and is thereafter divided into air that flows through the upper bypasses 301a, 301b and the lower bypasses 302a, 302b and air that passes through the heater core 5. The air joins at a rear side of the heater core 5, collides against the wall portion 2a of the air conditioning case 2 and the front wall 40a of the space defining wall member 40, and is changed in flowing direction.

Of this air, air that has collided against the front wall 40a of the space defining wall member 40 is partly turned to flow upward to the vent blowout openings 20a, 20b, and the rest of the air is turned to flow downward to the foot blowout openings 30a, 30b. The air flowing along the surface of the space defining wall member 40 to the vent blowout openings 20a, 20b causes the aspiration effect in the first driver's seat-side communicating portion 401a and the first passenger's seat-side communicating portion 401b. This makes the pressure in the first driver's seat-side communicating portion 401a and the first passenger's seat-side communicating portion 401b relatively lower than the pressure in the second driver's seat-side communicating portion 402a and the second passenger's seat-side communicating portion 402b so that air in the function addition space 41 is drawn in to the first communicating portions 401a, 401b, and that air in the respective divisional passages 3a, 3b (the mixing areas 303a, 303b) is taken in by suction to the function addition space 41 via the second communicating portions 402a, 402b. Thus, the air to which ions have been added in the function addition space 41 by the ion generator 44 can be supplied to the driver's seat-side vent blowout opening 20a and the passenger's seat-side vent blowout opening 20b.

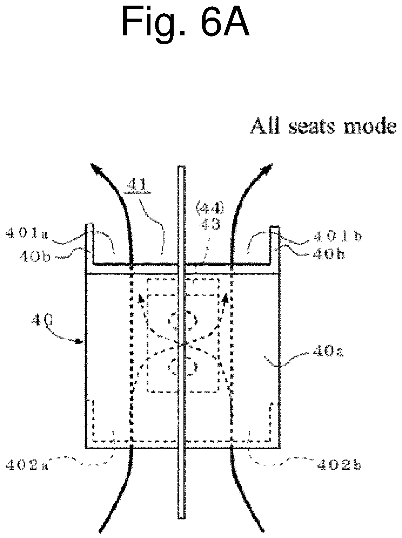

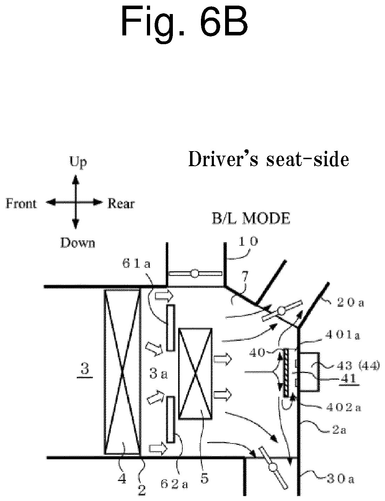

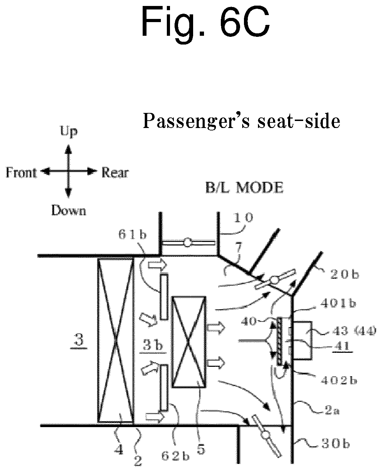

In this manner, in a blowout mode (all seats mode) in which the vent blowout openings at the driver's seat side and the passenger's seat side are open, the function addition space 41 is shared by the driver's seat-side divisional passage 3a and the passenger's seat-side divisional passage 3b. Consequently, as illustrated in Figs. 6A to 6C, the air that has been taken in by suction via the second driver's seat-side communicating portion 402a and the second passenger's seat-side communicating portion 402b is partly mixed in the function addition space 41. After ions are added to the air in the function addition space 41, the air does not stagnate but is supplied to the driver's seat-side vent blowout opening 20a via the first driver's seat-side communicating portion 401a and supplied to the passenger's seat-side vent blowout opening 20b via the first passenger's seat-side communicating portion 401b.

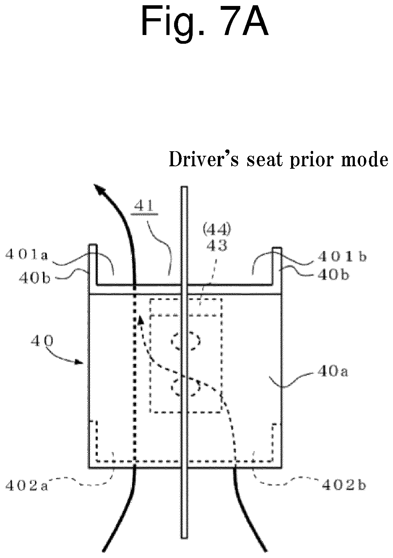

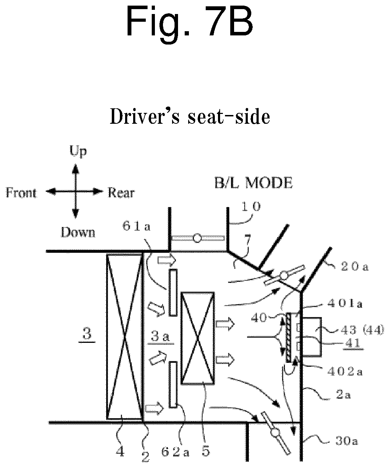

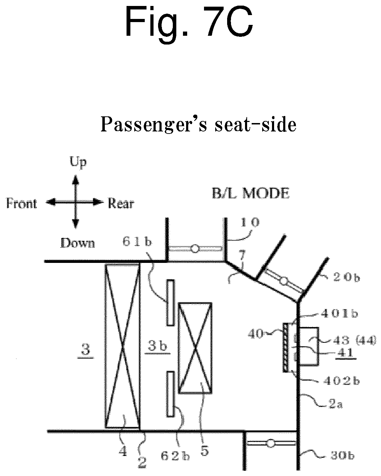

Even in a driver's seat prior mode in which conditioned air is only supplied to the driver's seat (a mode in which the openings at the passenger's seat side are all closed), the aspiration effect in the first driver's seat-side communicating portion 401a makes the first driver's seat-side communicating portion 401a relatively low in pressure. Consequently, as illustrated in Figs. 7A to 7C, via the second driver's seat-side communicating portion 402a and the second passenger's seat-side communicating portion 402b, the air in the driver's seat-side mixing area 303a and the passenger's seat-side mixing area 303b is taken in by suction to the function addition space 41 and drawn into the first driver's seat-side communicating portion 401a so that the ion-added air can be sufficiently supplied to the driver's seat-side vent blowout opening 20a.

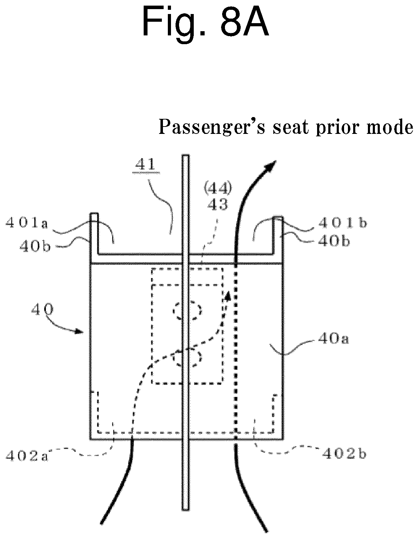

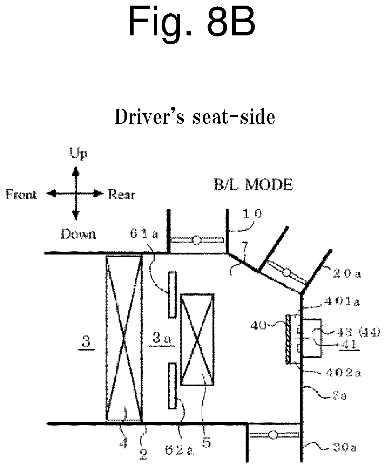

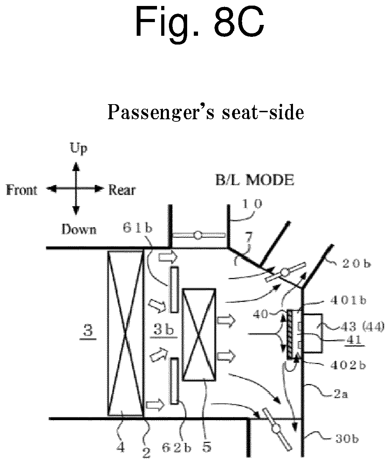

Similarly, even in a passenger's seat prior mode in which conditioned air is only supplied to the passenger's seat (a mode in which the openings at the driver's seat side are all closed), the aspiration effect in the first passenger's seat-side communicating portion 401b makes the first passenger's seat-side communicating portion 401b relatively low in pressure. Consequently, as illustrated in Figs. 8A to 8C, via the second driver's seat-side communicating portion 402a and the second passenger's seat-side communicating portion 402b, the air in the driver's seat-side mixing area 303a and the passenger's seat-side mixing area 303b is taken in by suction to the function addition space 41 and drawn into the first passenger's seat-side communicating portion 401b so that the ion-added air can be sufficiently supplied to the passenger's seat-side vent blowout opening 20b.

In this manner, no matter from which of the divisional passages the air is supplied to the cabin, the function addition space 41 is shared by the divisional passages 3a, 3b so that the air to which ions have been added by the single ion generator 44 can be sufficiently supplied to the cabin via any of the divisional passages.

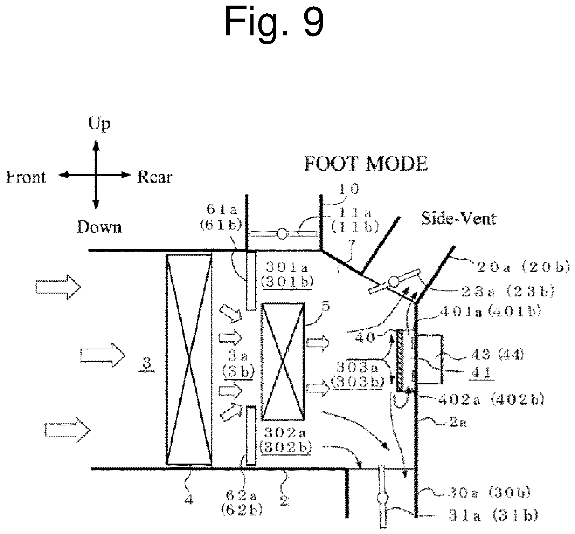

In a foot blowout mode, as illustrated in Fig. 9, the air mixing doors 6a, 6b occupy "full hot" positions (positions to cause the air that has passed through the evaporator 4 to pass through the heater core 5 alone), the defrost blowout opening 10 is closed, the center vent blowout openings 21 are closed, and the side vent blowout openings 22 and the foot blowout openings 30 are open. In the foot blowout mode, the space defining wall member 40 disposed in the vicinity of the side vent blowout openings 22 causes the aspiration effect in the first communicating portions (the first driver's seat-side communicating portion 401a and the first passenger's seat-side communicating portion 401b) to draw the air from the function addition space 41 so that the ion-added air can be supplied from the side vent blowout openings 22 to the cabin.

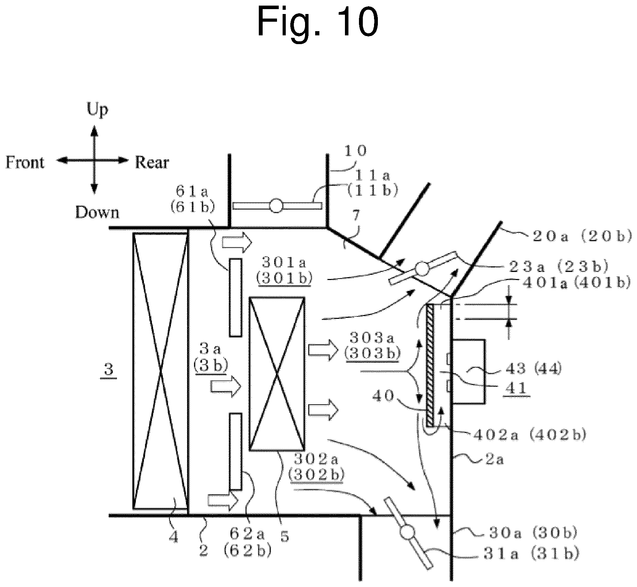

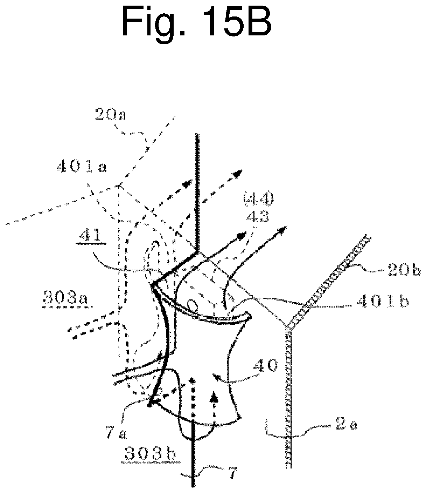

The configuration for supplying the ion-added air to the vent blowout openings 20 has been described so far. Modifications illustrated in Figs. 10 to 15A and 15B may be adopted as methods of more effectively supplying air to which ions are added in the respective divisional passages 3a, 3b to the vent blowout openings 20a, 20b.

Among these methods, in a modification illustrated in Fig. 10, a distance between the first communicating portion 401a, 401b and the vent blowout opening 20a, 20b is made shorter than a distance between the second communicating portion 402a, 402b and the foot blowout opening 30a, 30b. Also, the first communicating portions 401a, 401b are directly opened toward the vent blowout openings 20a, 20b so that the ion-added air is directly supplied to the vent blowout openings 20a, 20b.

With this configuration, direct communication of the first communicating portions 401a, 401b with the vent blowout openings 20a, 20b makes the aspiration effect easier to obtain so that the air to which ions have been added in the function addition space 41 can be more effectively supplied to the vent blowout openings 20a, 20b.

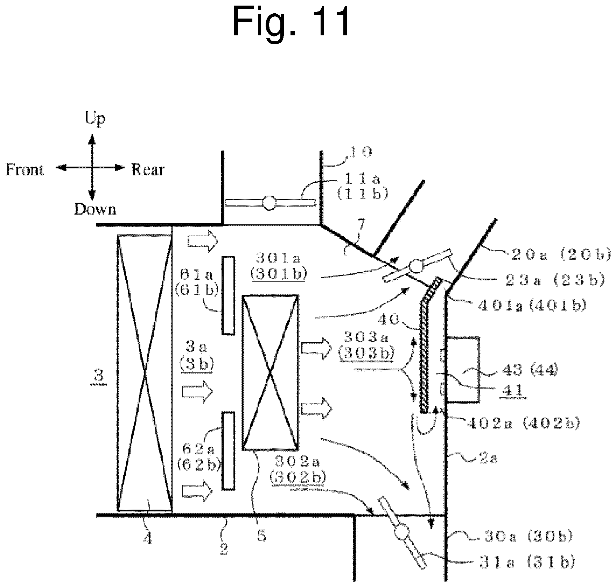

In the above-described configuration illustrated in Fig. 10, to further enhance the aspiration effect, as illustrated in Fig. 11, an upper end portion of the space defining wall member 40 may be extended along inner walls of the vent blowout openings 20a, 20b toward open ends so that the first communicating portions 401a, 401b are extended into the vent blowout openings 20.

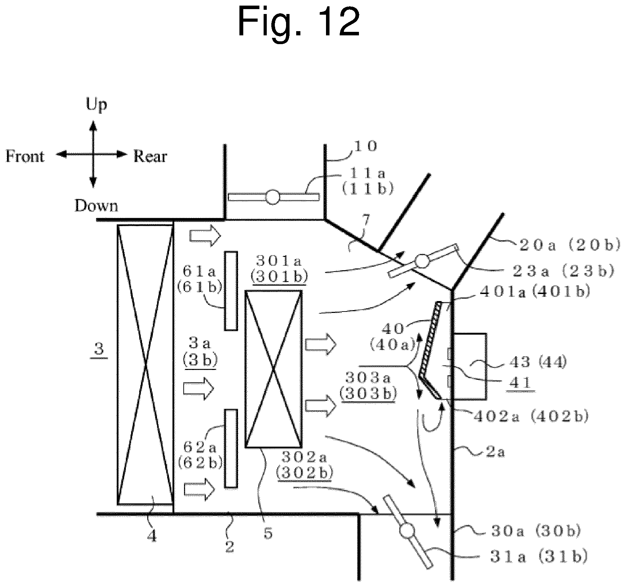

Alternatively, as illustrated in Fig. 12, the space defining wall member 40 may be formed in such a manner that an intermediate portion of the front wall 40a against which the air flowing from the upstream side collides protrudes toward the upstream side (the mixing areas 303a, 303b).

With this configuration, the air flowing from the upstream side is smoothly divided into an upward flow and a downward flow at the front wall 40a of the space defining wall member 40. Consequently, a region where the air stagnates in front of the space defining wall member 40 is less likely to be formed, and the air can be reliably oriented to flow upward and downward so that the aspiration effect in the communicating portions can be enhanced.

In this case as well, to enhance the aspiration effect in the first communicating portions 401a, 401b opened toward the vent blowout openings 20a, 20b, a position for dividing the air may be located in a lower portion of the front wall 40a to make most of the air that has collided against the front wall 40a flow toward the vent blowout openings 20a, 20b (upward) .

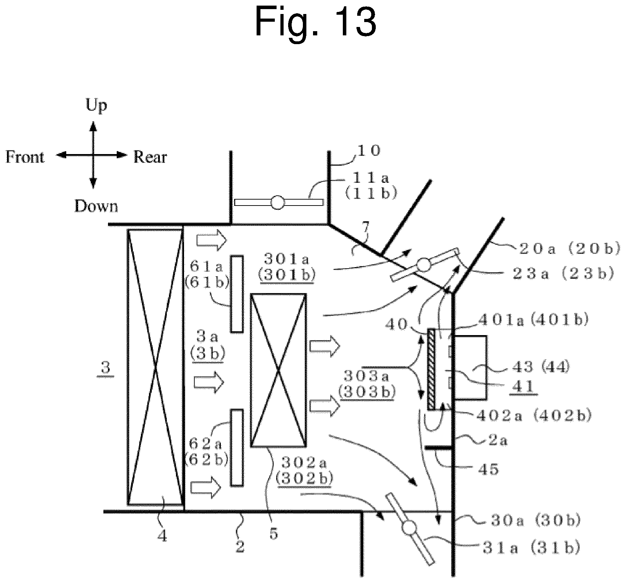

In a modification illustrated in Fig. 13, a plate-shaped guide portion 45 is disposed below the second communicating portions 402a, 402b of the space defining wall member 40 and protrudes inward from the wall portion 2a of the air conditioning case 2 so that the guide portion 45 receives air flowing downward from an upper side of the guide portion 45 to more efficiently introduce the air into the second communicating portions 402a, 402b.

With this configuration, even when the foot blowout openings 30 are open, the air immediately under the space defining wall member 40 is prevented from being introduced into the foot blowout openings 30 so as to prevent the air in the vicinity of the second communicating portions 402a, 402b from being inconveniently drawn downward and to enable promotion of suction of the air into the function addition space 41 by the aspiration effect in the first communicating portions 401a, 401b.

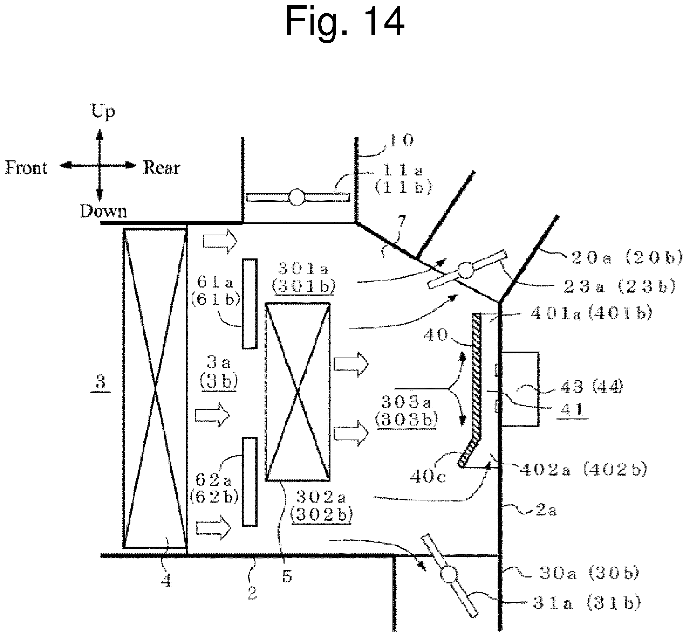

Alternatively, as illustrated in Fig. 14, an element to promote introduction of the air into the function addition space 41 may be provided for the space defining wall member 40. For example, a lower end portion of the space defining wall member 40 may be expanded toward the mixing areas 303a, 303b to form a guide portion 40c to increase an opening area in the lower end portion of the space defining wall member 40. This facilitates formation of air flows into the function addition space 41.

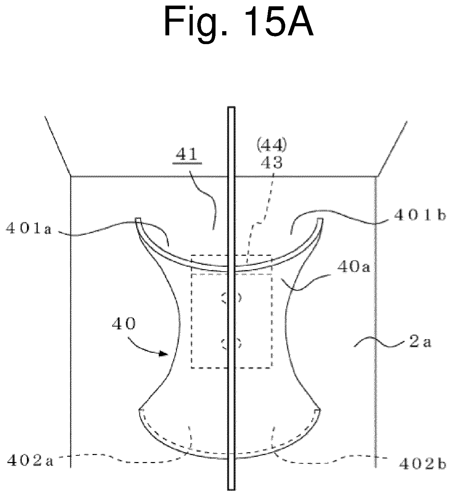

The above-described space defining wall member 40 has a substantially uniform passage cross-sectional area from the first communicating portions 401a, 401b to the second communicating portions 402a, 402b to make the function addition space 41 communicate with the air passage 3. However, as in a modification illustrated in Figs. 15A and 15B, the space defining wall member 40 may have an intermediate portion reduced in passage cross-sectional area (may have a smaller passage cross-sectional area than the communicating portions 401a, 401b and 402a, 402b on opposite ends). In the modification illustrated in Figs. 15A and 15B, the function addition space 41 has a semicircular cross-sectional shape, and the intermediate portion of the space defining wall member 40 is gradually recessed to make the intermediate portion have the minimum passage cross-sectional area.

This configuration promotes introduction of the air into the function addition space 41. Moreover, the air taken into the function addition space 41 by suction receives ions from the ion generator 44 and is increased in flowrate in the intermediate portion and thereafter discharged from the communicating portion (the first communicating portions 401a, 401b) having a larger passage cross-sectional area. Thus, without colliding against the space defining wall member 40, the ion-added air can be quickly supplied to the vent blowout openings 20a, 20b. This makes it possible to avoid inconvenience of the ion-added air being decreased in function (losing the ions) by colliding against the space defining wall member 40. Also, the ion-added air is increased in flowrate to be reliably supplied to the vent blowout openings 20a, 20b.

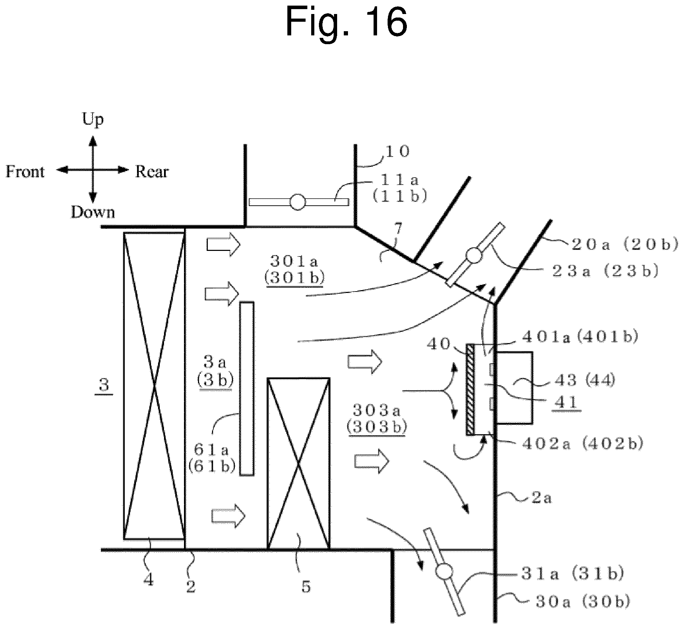

In the above-described configuration, the upper bypasses 301a, 301b are formed above the heater core 5 whereas the lower bypasses 302a, 302b are formed below the heater core 5, and the opening degrees of the bypasses 301a, 301b, 302a, 302b are adjusted by the different air mixing doors 61a, 61b, 62a, 62b. However, as illustrated in Fig. 16, in each of the divisional passages 3a, 3b, the bypass 301a, 301b may be only disposed above the heater core 5, and a ratio of the air passing through the bypass 301a, 301b to the air passing through the heater core 5 may be adjusted by the single air mixing door 61a, 61b. With such an air conditioner, substantially the same configuration can produce substantially the same functions and effects.

In the above-described examples, the ion generator 44 is disposed outside of the wall portion 2a of the air conditioning case 2. However, the ion generator 44 may be disposed inside of the wall portion 2a or on the space defining wall member 40.

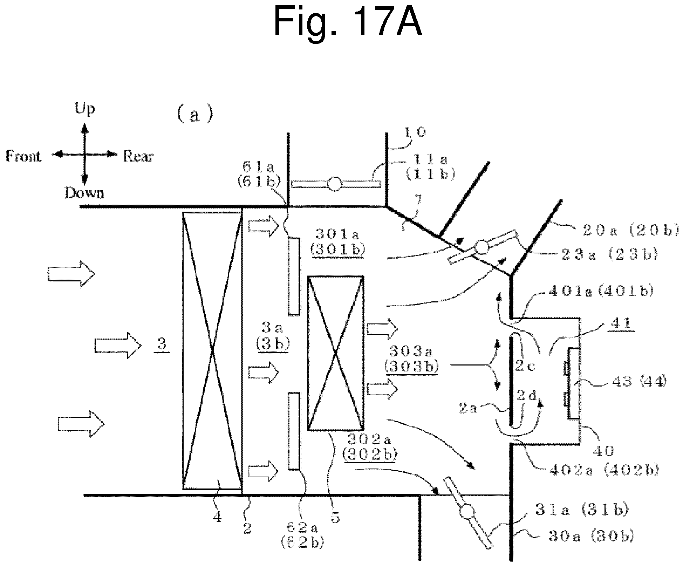

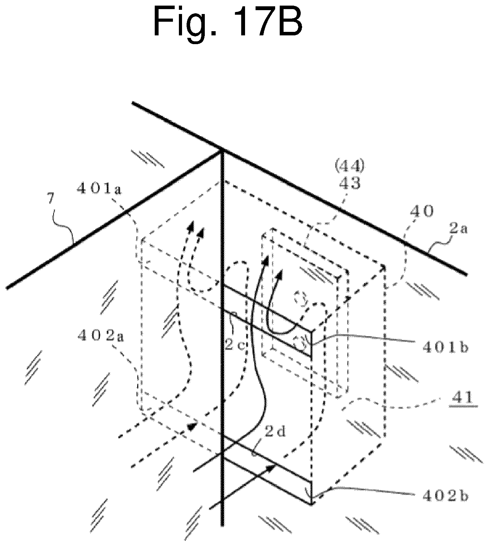

In the above-described examples, the space defining wall member 40 is disposed inside (on an inner surface of the wall portion 2a) of the air conditioning case 2. However, as in a modification illustrated in Figs. 17A and 17B, the space defining wall member 40 may be disposed outside (on an outer surface of the wall portion 2a) of the air conditioning case 2, and the function adding device 43 (the ion generator 44) may be disposed on the wall portion 2a of the air conditioning case 2 covered with the space defining wall member 40 or on the space defining wall member 40.

In the modification illustrated in Figs. 17A and 17B, the space defining wall member 40 has a box shape with a portion opposed to the wall portion 2a of the air conditioning case 2 being opened. The space defining wall member 40 is disposed outside of the wall portion 2a of the air conditioning case 2 while crossing the partition wall 7 so that the function addition space 41 is formed between an inner surface of the space defining wall member 40 and the wall portion 2a. The ion generator 44 is secured to the inner surface of the space defining wall member 40 opposed to the wall portion 2a of the air conditioning case 2 so that ions can be added to the air in the function addition space 41.

The wall portion 2a of the air conditioning case 2 covered with the space defining wall member 40 has openings 2c and 2d facing an upper end portion and a lower end portion of the function addition space 41 and crossing the partition wall 7. Of the opening 2c facing the upper end portion of the function addition space 41, a portion opened toward the driver's seat-side divisional passage 3a forms the first driver's seat-side communicating portion 401a that allows the function addition space 41 to communicate with the driver's seat-side divisional passage 3a and is opened closer to the driver's seat-side vent blowout opening 20a whereas a portion opened toward the passenger's seat-side divisional passage 3b forms the first passenger's seat-side communicating portion 401b that allows the function addition space 41 to communicate with the passenger's seat-side divisional passage 3b and is opened closer to the passenger's seat-side vent blowout opening 20b.

Of the opening 2d facing the lower end portion of the function addition space 41, a portion opened toward the driver's seat-side divisional passage 3a forms the second driver's seat-side communicating portion 402a that allows the function addition space 41 to communicate with the driver's seat-side divisional passage 3a and is opened closer to the driver's seat-side foot blowout opening 30a whereas a portion opened toward the passenger's seat-side divisional passage 3b forms the second passenger's seat-side communicating portion 402b that allows the function addition space 41 to communicate with the passenger's seat-side divisional passage 3b and is opened closer to the passenger' s seat-side foot blowout opening 30b.

In this configuration, when the air that has collided against the wall portion 2a of the air conditioning case 2 and changed in flowing direction toward the vent blowout openings 20a, 20b passes before the first communicating portions 401a, 401b, the aspiration effect is generated in the first communicating portions 401a, 401b, thereby making the static pressure in the first communicating portions 401a, 401b relatively lower than the static pressure in the second communicating portions 402a, 402b. Consequently, in each of the divisional passages 3a, 3b, after the air in the mixing area 303a, 303b is partly taken into the function addition space 41 by suction via the second communicating portion 402a, 402b and receives ions from the ion generator 44 in the function addition space 41, the air is returned to the mixing area 303a, 303b via the first communicating portion 401a, 401b and flows toward the vent blowout opening 20a, 20b. This makes it possible to sufficiently supply the ion-added air to the vent blowout opening 20a, 20b from each of the divisional passages 3a, 3b.

In the above-described examples, the air to which ions have been added in the function addition space 41 is supplied via the vent blowout openings 20a, 20b. However, when there is need to supply the ion-added air via the foot blowout openings 30a, 30b, an installation position and a shape of the space defining wall member 40 may be adjusted to make the second communicating portions 402a, 402b in the vicinity of the foot blowout openings 30a, 30b lower in pressure than the first communicating portions 401a, 401b in a blowout mode (a defrost-foot blowout mode, the foot blowout mode) in which the foot blowout openings 30a, 30b are open.

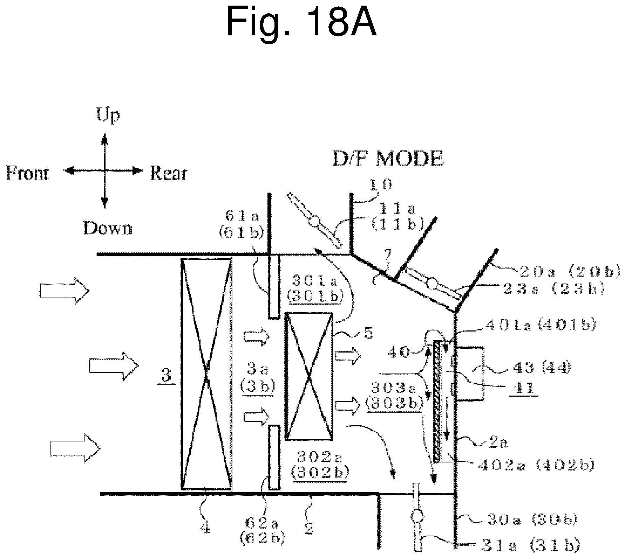

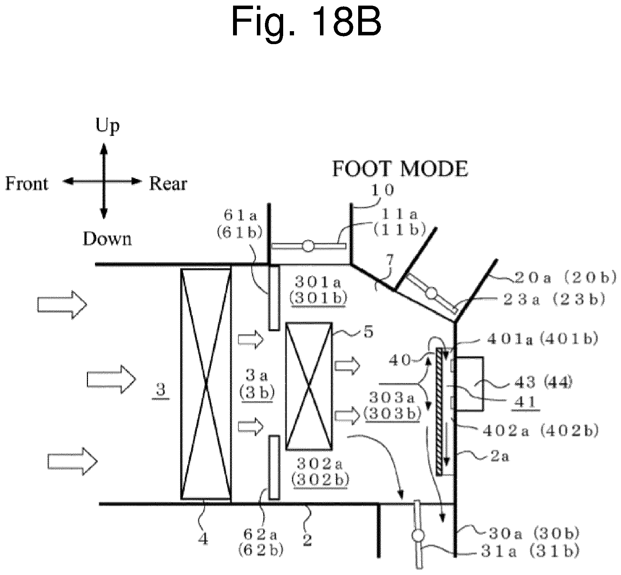

For example, as illustrated in Figs. 18A and 18B, the second communicating portions 402a, 402b of the space defining wall member 40 that are opened toward the foot blowout openings 30a, 30b are extended downward and closer to the foot blowout openings 30a, 30b so as to enhance the aspiration effect in the second communicating portions 402a, 402b.

As modifications of the example illustrated in Figs. 18A and 18B, the various configurations described above to supply ions to the vent blowout openings 20a, 20b may be similarly adopted in accordance with the configuration illustrated in Figs. 18A and 18B. For example, a guide portion may be disposed immediately above the first communicating portions 401a, 401b, an intermediate portion of the front wall 40a may be protruded upstream to increase the air divided in front of the space defining wall member 40 and made to flow to the foot blowout openings 30a, 30b, and an upper end portion of the space defining wall member 40 may be extended toward the mixing areas 303a, 303b.

In the above-described examples, the ion generator 44 to add ions to air is given as an example of the function adding device 43 to add a function to the air that has passed through the function addition space 41. However, the ion generator 44 may be replaced with any of a fragrance generator to add a fragrance function, a deodorizer to add a deodorizing function, a humidity controller to add a humidity control function, and a sterilizer to add a sterilizing function or may be combined with these devices as suited.

In the above-described examples, the vehicular air conditioner includes the air passage divided into the left and right divisional passages by the partition wall to enable individual control of temperatures in the left and right divisional passages. However, in the case of dividing the air passage into upper and lower divisional passages by the partition wall and in the case of dividing the air passage into three or more divisional passages, the space defining wall member 40 may be disposed across the partition wall 7 to make the function addition space 41 shared by a plurality of divisional passages.

### Description of Reference Numerals and Signs

1: vehicular air conditioner
2: air conditioning case
2a: wall portion
3: air passage
3a: driver's seat-side divisional passage
3b: passenger's seat-side divisional passage
4: evaporator
5: heater core
61a, 61b, 62a, 62b: air mixing door
20a: driver's seat-side vent blowout opening
20b: passenger's seat-side vent blowout opening
30a: driver's seat-side foot blowout opening
30b: passenger's seat-side foot blowout opening
40: space defining wall member
41: function addition space
401a: first driver's seat-side communicating portion
401b: first passenger's seat-side communicating portion
402a: second driver's seat-side communicating portion
402b: second passenger's seat-side communicating portion
43: function adding device
44: ion generator

## Claims

1. A vehicular air conditioner (1), when mounted in a vehicle, configured to blow air to which a function has been added into a cabin, the air conditioner (1) comprising:
an air conditioning case (2) comprising:
an internal air passage (3); and
a plurality of blowout openings (10, 20a, 20b, 30a, 30b) capable of supplying intake air into the cabin; and
a heat exchanger (4, 5) disposed in the air conditioning case (2) and configured to perform heat exchange of the intake air,
the air passage (3) at least downstream of the heat exchanger (4) being divided into a plurality of divisional passages (3a, 3b) by a partition wall (7),
**characterized in that** a space defining wall member (40) is disposed on a wall portion (2a) of the air conditioning case (2), the wall portion (2a) being configured to change a flowing direction of the air that has passed through the heat exchanger (4, 5), the space defining wall member (40) being configured to define a function addition space (41) between the space defining wall member (40) and the wall portion (2a), the function addition space (41) being not divided by the partition wall (7),
wherein a function adding device (43) is disposed on the wall portion (2a) or the space defining wall member (40) and configured to add a predetermined function to the air flowing through the function addition space (41),
wherein the function addition space (41) is configured to communicate with each of the divisional passages (3a, 3b) via at least two communicating portions (401a, 401b, 402a, 402b), and
wherein at least one of the communicating portions (401a, 401b) in each of the divisional passages (3a, 3b) is configured to be opened toward one of the blowout openings (10, 20a, 20b, 30a, 30b) so that a pressure in the at least one of the communicating portions (401a, 401b) is lower than a pressure in another of the communicating portions (402a, 402b) in a predetermined blowout mode,
wherein the plurality of blowout openings comprise
a vent blowout opening (20a, 20b) configured to supply air blown toward an upper portion of the cabin, and
a foot blowout opening (30a, 30b) configured to supply air blown toward a lower portion of the cabin,
wherein the space defining wall member (40) is disposed on the wall portion (2a) between the vent blowout opening (20a, 20b) and the foot blowout opening (30a, 30b),
wherein the partition wall (7) is configured to divide the air passage (3) at least downstream of the heat exchanger (4) into a driver's seat-side divisional passage (3a) and a passenger's seat-side divisional passage (3b), divide the vent blowout opening into a driver's seat-side vent blowout opening (20a) and a passenger's seat-side vent blowout opening (20b), and divide the foot blowout opening into a driver's seat-side foot blowout opening (30a) and a passenger's seat-side foot blowout opening (30b),
wherein the function addition space (41) is configured to communicate with the driver's seat-side divisional passage (3a) via a first driver's seat-side communicating portion (401a) disposed close to the driver's seat-side vent blowout opening (20a) and a second driver's seat-side communicating portion (402a) disposed close to the driver's seat-side foot blowout opening (30a), and the function addition space (41) is configured to communicate with the passenger's seat-side divisional passage (3b) via a first passenger's seat-side communicating portion (401b) disposed close to the passenger's seat-side vent blowout opening (20b) and a second passenger's seat-side communicating portion (402b) disposed close to the passenger's seat-side foot blowout opening (30b),.

2. The vehicular air conditioner (1) according to claim 1,
wherein in a blowout mode in which the driver's seat-side vent blowout opening (20a) is open, the first driver's seat-side communicating portion (401a) is able to be opened toward the driver's seat-side vent blowout opening (20a) to make the first driver's seat-side communicating portion (401a) have a lower pressure than the second driver's seat-side communicating portion (402a), and in a blowout mode in which the passenger's seat-side vent blowout opening (20b) is open, the first passenger's seat-side communicating portion (401b) is able to be opened toward the passenger's seat-side vent blowout opening (20b) to make the first passenger's seat-side communicating portion (401b) have a lower pressure than the second passenger's seat-side communicating portion (402b).

3. The vehicular air conditioner (1) according to claim 1,
wherein in a blowout mode in which the driver's seat-side foot blowout opening (30a) is open, the second driver's seat-side communicating portion (402a) is able to be opened toward the driver's seat-side foot blowout opening (30a) to make the second driver's seat-side communicating portion (402a) have a lower pressure than the first driver's seat-side communicating portion (401a), and in a blowout mode in which the passenger's seat-side foot blowout opening (30b) is open, the second passenger's seat-side communicating portion (402b) is able to be opened toward the passenger's seat-side foot blowout opening (30b) to make the second passenger's seat-side communicating portion (402b) have a lower pressure than the first passenger's seat-side communicating portion (401b).

4. The vehicular air conditioner (1) according to any one of claims 1 to 3, wherein in each of the divisional passages (3a, 3b), the communicating portion (401a, 401b) relatively low in pressure is formed closer to the blowout opening than the other of the communicating portions (402a, 402b) is to the blowout opening.

5. The vehicular air conditioner (1) according to any one of claims 1 to 4, wherein in the divisional passages (3a, 3b), the space defining wall member (40) is disposed inside of the wall portion (2a) and comprises a surface against which the air flowing from an upstream side collides, the surface comprising an intermediate portion protruding toward the upstream side.

6. The vehicular air conditioner (1) according to any one of claims 1 to 4, wherein the space defining wall member (40) is disposed outside of the wall portion (2a) of the air conditioning case (2).

7. The vehicular air conditioner (1) according to any one of claims 1 to 5, wherein in the divisional passages (3a, 3b) , a guide portion (45, 40c) is disposed on or in a vicinity of a portion of the space defining wall member (40) where the other of the communicating portions (402a, 402b) is formed, the guide portion (45, 40c) being configured to promote introduction of the air into the other of the communicating portions (402a, 402b).

8. The vehicular air conditioner (1) according to any one of claims 1 to 7, wherein the function addition space (41) defined by the space defining wall member (40) comprises an intermediate portion having a passage cross-sectional area smaller than the communicating portions.

9. The vehicular air conditioner (1) according to any one of claims 1 to 8, wherein the heat exchanger (4, 5) comprises
a cooling heat exchanger (4) configured to cool the air passing therethrough, and
a heating heat exchanger (5) disposed downstream of the cooling heat exchanger (4) and configured to heat the air passing therethrough, the heating heat exchanger (5) being arranged in the air passage (3) in such a manner that bypasses (301a, 301b, 302a, 302b) are formed above and below the heating heat exchanger (5).

10. The vehicular air conditioner (1) according to any one of claims 1 to 8, wherein the heat exchanger (4, 5) comprises
a cooling heat exchanger (4) configured to cool the air passing therethrough, and
a heating heat exchanger (5) disposed downstream of the cooling heat exchanger (4) and configured to heat the air passing therethrough, the heating heat exchanger (5) being arranged in the air passage (3) in such a manner that bypasses (301a, 301b) are formed only above the heating heat exchanger (5) .

11. The vehicular air conditioner (1) according to any one of claims 1 to 10, wherein the function added to the air by the function adding device (43) comprises at least one of an ion adding function, a fragrance function, a deodorizing function, a humidity control function, and a sterilizing function.

## Patentansprüche

1. Fahrzeugklimaanlage (1), die, wenn sie in einem Fahrzeug angebracht ist, dazu ausgelegt ist, Luft, der eine Funktion hinzugefügt wurde, in einen Innenraum zu blasen, wobei die Klimaanlage (1) Folgendes umfasst:
ein Klimaanlagengehäuse (2), umfassend:
einen inneren Luftdurchlass (3); und
eine Vielzahl von Ausblasöffnungen (10, 20a, 20b, 30a, 30b), die in der Lage sind, Ansaugluft in die Kabine zu leiten; und
einen Wärmetauscher (4, 5), der in dem Klimaanlagengehäuse (2) angeordnet und dazu ausgelegt ist, einen Wärmeaustausch der Ansaugluft durchzuführen,
wobei der Luftdurchlass (3) mindestens stromabwärts des Wärmetauschers (4) durch eine Trennwand (7) in eine Vielzahl von Teilungsdurchlässen (3a, 3b) unterteilt ist,
**dadurch gekennzeichnet, dass** ein raumdefinierendes Wandelement (40) an einem Wandabschnitt (2a) des Klimaanlagengehäuses (2) angeordnet ist, wobei der Wandabschnitt (2a) dazu ausgelegt ist, eine Strömungsrichtung der Luft zu ändern, die durch den Wärmetauscher (4, 5) geströmt ist, wobei das raumdefinierende Wandelement (40) dazu ausgelegt ist, einen Funktionszusatzraum (41) zwischen dem raumdefinierenden Wandelement (40) und dem Wandabschnitt (2a) zu definieren, wobei der Funktionszusatzraum (41) nicht durch die Trennwand (7) unterteilt ist,
wobei eine Funktionszusatzvorrichtung (43) an dem Wandabschnitt (2a) oder dem raumdefinierenden Wandelement (40) angeordnet und dazu ausgelegt ist, der durch den Funktionszusatzraum (41) strömenden Luft eine vorbestimmte Funktion hinzuzufügen,
wobei der Funktionszusatzraum (41) dazu ausgelegt ist, mit jedem der Teilungsdurchlässe (3a, 3b) über mindestens zwei kommunizierende Abschnitte (401a, 401b, 402a, 402b) zu kommunizieren, und
wobei mindestens einer der kommunizierenden Abschnitte (401a, 401b) in jedem der Teilungsdurchlässe (3a, 3b) dazu ausgelegt ist, zu einer der Ausblasöffnungen (10, 20a, 20b, 30a, 30b) hin geöffnet zu werden, sodass in einem vorbestimmten Ausblasmodus ein Druck in dem mindestens einen der kommunizierenden Abschnitte (401a, 401b) niedriger als ein Druck in einem anderen der kommunizierenden Abschnitte (402a, 402b) ist,
wobei die Vielzahl von Ausblasöffnungen Folgendes umfasst:
eine Lüftungsausblasöffnung (20a, 20b), die dazu ausgelegt ist, Luft zuzuführen, die in einen oberen Abschnitt der Kabine geblasen wird, und
eine Fußausblasöffnung (30a, 30b), die dazu ausgelegt ist, Luft zuzuführen, die in einen unteren Abschnitt der Kabine geblasen wird,
wobei das raumdefinierende Wandelement (40) an dem Wandabschnitt (2a) zwischen der Lüftungsausblasöffnung (20a, 20b) und der Fußausblasöffnung (30a, 30b) angeordnet ist,
wobei die Trennwand (7) dazu ausgelegt ist, den Luftdurchlass (3) mindestens stromabwärts des Wärmetauschers (4) in einen fahrersitzseitigen Teilungsdurchlass (3a) und einen beifahrersitzseitigen Teilungsdurchlass (3b) zu unterteilen, die Lüftungsausblasöffnung in eine fahrersitzseitige Lüftungsausblasöffnung (20a) und eine beifahrersitzseitige Lüftungsausblasöffnung (20b) zu unterteilen und die Fußausblasöffnung in eine fahrersitzseitige Fußausblasöffnung (30a) und eine beifahrersitzseitige Fußausblasöffnung (30b) zu unterteilen,
wobei der Funktionszusatzraum (41) dazu ausgelegt ist, über einen ersten fahrersitzseitigen Kommunikationsabschnitt (401a), der nahe der fahrersitzseitigen Lüftungsausblasöffnung (20a) angeordnet ist, und einen zweiten fahrersitzseitigen Kommunikationsabschnitt (402a), der nahe der fahrersitzseitigen Fußausblasöffnung (30a) angeordnet ist, mit dem fahrerseitigen Teilungsdurchlass (3a) zu kommunizieren, und der Funktionszusatzraum (41) dazu ausgelegt ist, mit dem beifahrerseitigen Teilungsdurchlass (3b) über einen ersten beifahrerseitigen Kommunikationsabschnitt (401b), der nahe der beifahrerseitigen Lüftungsausblasöffnung (20b) angeordnet ist, und einen zweiten beifahrerseitigen Kommunikationsabschnitt (402b), der nahe der beifahrerseitigen Fußausblasöffnung (30b) angeordnet ist, zu kommunizieren.

2. Fahrzeugklimaanlage (1) gemäß Anspruch 1,
wobei in einem Ausblasmodus, in dem die fahrersitzseitige Lüftungsausblasöffnung (20a) geöffnet ist, der erste fahrersitzseitige Kommunikationsabschnitt (401a) in Richtung der fahrersitzseitigen Lüftungsausblasöffnung (20a) geöffnet werden kann, damit der erste fahrersitzseitige kommunizierende Abschnitt (401a) einen niedrigeren Druck als der zweite fahrersitzseitige kommunizierende Abschnitt (402a) aufweist, und in einem Ausblasmodus, in dem die beifahrerseitige Lüftungsausblasöffnung (20b) geöffnet ist, der erste beifahrerseitige kommunizierende Abschnitt (401b) in Richtung der beifahrerseitigen Lüftungsausblasöffnung (20b) geöffnet werden kann, damit der erste beifahrerseitige kommunizierende Abschnitt (401b) einen niedrigeren Druck als der zweite beifahrerseitige kommunizierende Abschnitt (402b) aufweist.

3. Fahrzeugklimaanlage (1) gemäß Anspruch 1,
wobei in einem Ausblasmodus, in dem die fahrersitzseitige Fußausblasöffnung (30a) geöffnet ist, der zweite fahrersitzseitige kommunizierende Abschnitt (402a) in Richtung der fahrersitzseitigen Fußausblasöffnung (30a) geöffnet werden kann, damit der zweite fahrersitzseitige kommunizierende Abschnitt (402a) einen niedrigeren Druck als der erste fahrersitzseitige kommunizierende Abschnitt (401a) aufweist, und in einem Ausblasmodus, in dem die beifahrerseitige Fußausblasöffnung (30b) geöffnet ist, der zweite beifahrerseitige kommunizierende Abschnitt (402b) in Richtung der beifahrerseitigen Fußausblasöffnung (30b) geöffnet werden kann, damit der zweite beifahrerseitige kommunizierende Abschnitt (402b) einen niedrigeren Druck als der erste beifahrerseitige kommunizierende Abschnitt (401b) aufweist.

4. Fahrzeugklimaanlage (1) gemäß einem der Ansprüche 1 bis 3, wobei in jedem der Teilungsdurchlässe (3a, 3b) der kommunizierende Abschnitt (401a, 401b) mit relativ niedrigem Druck näher an der Ausblasöffnung als der andere der kommunizierenden Abschnitte (402a, 402b) an der Ausblasöffnung ausgebildet ist.

5. Fahrzeugklimaanlage (1) gemäß einem der Ansprüche 1 bis 4, wobei in den Teilungsdurchlässen (3a, 3b) das raumdefinierende Wandelement (40) innerhalb des Wandabschnitts (2a) angeordnet ist und eine Fläche umfasst, gegen die die von einer stromaufwärts gelegenen Seite strömende Luft prallt, wobei die Fläche einen Zwischenabschnitt umfasst, der in Richtung der stromaufwärts gelegenen Seite vorsteht.

6. Fahrzeugklimaanlage (1) gemäß einem der Ansprüche 1 bis 4, wobei das raumdefinierende Wandelement (40) außerhalb des Wandabschnitts (2a) des Klimaanlagengehäuses (2) angeordnet ist.

7. Fahrzeugklimaanlage (1) gemäß einem der Ansprüche 1 bis 5, wobei in den Teilungsdurchlässen (3a, 3b) ein Führungsabschnitt (45, 40c) an oder in der Nähe eines Abschnitts des raumdefinierenden Wandelements (40) angeordnet ist, an dem der andere der kommunizierenden Abschnitte (402a, 402b) ausgebildet ist, wobei der Führungsabschnitt (45, 40c) dazu ausgelegt ist, die Einleitung der Luft in den anderen der kommunizierenden Abschnitte (402a, 402b) zu fördern.

8. Fahrzeugklimaanlage (1) gemäß einem der Ansprüche 1 bis 7, wobei der durch das raumdefinierende Wandelement (40) definierte Funktionszusatzraum (41) einen Zwischenabschnitt mit einer Durchlassquerschnittsfläche aufweist, die kleiner als die kommunizierenden Abschnitte ist.

9. Fahrzeugklimaanlage (1) gemäß einem der Ansprüche 1 bis 8, wobei der Wärmetauscher (4, 5) Folgendes umfasst:
einen Kühlungswärmetauscher (4), der zum Kühlen der hindurchströmenden Luft ausgelegt ist, und
einen Heizungswärmetauscher (5), der stromabwärts des Kühlungswärmetauschers (4) angeordnet und dazu ausgelegt ist, die hindurchströmende Luft zu erwärmen, wobei der Heizungswärmetauscher (5) in dem Luftdurchlass (3) derart angeordnet ist, dass oberhalb und unterhalb des Heizungswärmetauschers (5) Umgehungsleitungen (301a, 301b, 302a, 302b) ausgebildet sind.

10. Fahrzeugklimaanlage (1) gemäß einem der Ansprüche 1 bis 8, wobei der Wärmetauscher (4, 5) Folgendes umfasst:
einen Kühlungswärmetauscher (4), der zum Kühlen der hindurchströmenden Luft ausgelegt ist, und
einen Heizungswärmetauscher (5), der stromabwärts des Kühlungswärmetauschers (4) angeordnet und dazu ausgelegt ist, die hindurchströmende Luft zu erwärmen, wobei der Heizungswärmetauscher (5) in dem Luftdurchlass (3) derart angeordnet ist, dass oberhalb und unterhalb des Heizungswärmetauschers (5) Umgehungsleitungen (301a, 301b) ausgebildet sind.

11. Fahrzeugklimaanlage (1) gemäß einem der Ansprüche 1 bis 10, wobei die der Luft durch die Funktionszusatzvorrichtung (43) hinzugefügte Funktion mindestens eine einer Ionenzugabefunktion, einer Duftfunktion, einer Desodorierungsfunktion, einer Feuchtigkeitssteuerungsfunktion und einer Sterilisierungsfunktion umfasst.

## Revendications

1. Climatiseur de véhicule (1), lorsque monté dans un véhicule, configuré pour souffler de l'air auquel une fonction a été ajoutée jusque dans une cabine, le climatiseur (1) comprenant :
un boîtier de climatisation (2) comprenant :
un passage d'air interne (3) ; et
une pluralité d'ouvertures de soufflerie (10, 20a, 20b, 30a, 30b) aptes à fournir de l'air d'admission jusque dans la cabine ; et
un échangeur thermique (4, 5) disposé dans le boîtier de climatisation (2) et configuré pour réaliser un échange thermique de l'air d'admission,
le passage d'air (3) au moins en aval de l'échangeur thermique (4) étant divisé en une pluralité de passages divisionnaires (3a, 3b) par une paroi de séparation (7), **caractérisé en ce qu'**un élément de paroi définissant un espace (40) est disposé sur une portion de paroi (2a) du boîtier de climatisation (2), la portion de paroi (2a) étant configurée pour changer une direction d'écoulement de l'air qui est passé à travers l'échangeur thermique (4, 5), l'élément de paroi définissant un espace (40) étant configuré pour définir un espace d'ajout de fonction (41) entre l'élément de paroi définissant un espace (40) et la portion de paroi (2a), l'espace d'ajout de fonction (41) n'étant pas divisé par la paroi de séparation (7),
un dispositif d'ajout de fonction (43) étant disposé sur la portion de paroi (2a) ou l'élément de paroi définissant un espace (40) et configuré pour ajouter une fonction prédéterminée à l'air s'écoulant à travers l'espace d'ajout de fonction (41),
l'espace d'ajout de fonction (41) étant configuré pour communiquer avec chacun des passages divisionnaires (3a, 3b) par l'intermédiaire d'au moins deux portions de communication (401a, 401b, 402a, 402b), et
au moins une des portions de communication (401a, 401b) dans chacun des passages divisionnaires (3a, 3b) étant configurée pour être ouverte vers l'une des ouvertures de soufflerie (10, 20a, 20b, 30a, 30b) de sorte qu'une pression dans l'au moins une des portions de communication (401a, 401b) soit plus basse qu'une pression dans une autre des portions de communication (402a, 402b) dans un mode de soufflerie prédéterminé,
la pluralité d'ouvertures de soufflerie comprenant
une ouverture de soufflerie en évent (20a, 20b) configurée pour fournir de l'air soufflé vers une portion plus haute de la cabine, et
une ouverture de soufflerie de pieds (30a, 30b) configurée pour fournir de l'air soufflé vers une portion plus basse de la cabine,
l'élément de paroi définissant un espace (40) étant disposé sur la portion de paroi (2a) entre l'ouverture de soufflerie en évent (20a, 20b) et l'ouverture de soufflerie de pieds (30a, 30b),
la paroi de séparation (7) étant configurée pour diviser le passage d'air (3) au moins en aval de l'échangeur thermique (4) en un passage divisionnaire côté siège de conducteur (3a) et un passage divisionnaire côté siège de passager (3b), diviser l'ouverture de soufflerie en évent en une ouverture de soufflerie en évent côté siège de conducteur (20a) et une ouverture de soufflerie en évent côté siège de passager (20b), et diviser l'ouverture de soufflerie de pieds en une ouverture de soufflerie de pieds côté siège de conducteur (30a) et une ouverture de soufflerie de pieds côté siège de passager (30b),
l'espace d'ajout de fonction (41) étant configuré pour communiquer avec le passage divisionnaire côté siège du conducteur (3a) par l'intermédiaire d'une première portion de communication côté siège du conducteur (401a) disposée à proximité de l'ouverture de soufflerie en évent côté siège du conducteur (20a) et une deuxième portion de communication côté siège du conducteur (402a) disposée à proximité de l'ouverture de soufflerie de pieds côté siège du conducteur (30a), et l'espace d'ajout de fonction (41) étant configuré pour communiquer avec le passage divisionnaire côté siège de passager (3b) par l'intermédiaire d'une première portion de communication côté siège de passager (401b) disposée à proximité de l'ouverture de soufflerie en évent côté siège de passager (20b) et d'une deuxième portion de communication côté siège de passager (402b) disposée à proximité de l'ouverture de soufflerie de pieds côté siège de passager (30b) .

2. Climatiseur de véhicule (1) selon la revendication 1,
dans un mode de soufflerie dans lequel l'ouverture de soufflerie en évent côté siège du conducteur (20a) est ouverte, la portion de communication côté siège du premier conducteur (401a) étant apte à être ouverte vers l'ouverture de soufflerie en évent côté siège du conducteur (20a) pour faire en sorte que la portion de communication côté siège du premier conducteur (401a) ait une pression plus basse que la portion de communication côté siège du deuxième conducteur (402a), et dans un mode de soufflerie dans lequel l'ouverture de soufflerie en évent côté siège du passager (20b) est ouverte, la portion de communication côté siège du premier passager (401b) étant apte à être ouverte vers l'ouverture de soufflerie en évent côté siège du passager (20b) pour faire en sorte que la portion de communication côté siège du premier passager (401b) à avoir une pression plus basse que la portion de communication côté siège du deuxième passager (402b).

3. Climatiseur de véhicule (1) selon la revendication 1,
dans un mode de soufflerie dans lequel l'ouverture de soufflerie de pieds côté siège du conducteur (30a) est ouverte, la portion de communication côté siège du deuxième conducteur (402a) étant apte à être ouverte vers l'ouverture de soufflerie de pieds côté siège du conducteur (30a) pour faire en sorte que la portion de communication côté siège du deuxième conducteur (402a) ait une pression plus basse que la portion de communication côté siège du premier conducteur (401a), et dans un mode de soufflerie dans lequel l'ouverture de soufflerie de pieds côté siège du passager (30b) est ouverte, la portion de communication côté siège du deuxième passager (402b) étant apte à être ouverte vers l'ouverture de soufflerie de pieds côté siège du passager (30b) pour faire en sorte que la portion de communication côté siège du deuxième passager (402b) ait une pression plus basse que la portion de communication côté siège du premier passager (401b).

4. Climatiseur de véhicule (1) selon l'une quelconque des revendications 1 à 3, dans lequel dans chacun des passages divisionnaires (3a, 3b), la portion de communication (401a, 401b) relativement basse en pression est formée plus près de l'ouverture de soufflerie que l'autre des portions de communication (402a, 402b) ne l'est de l'ouverture de soufflerie.

5. Climatiseur de véhicule (1) selon l'une quelconque des revendications 1 à 4, dans lequel dans les passages divisionnaire (3a, 3b), l'élément de paroi définissant un espace (40) est disposé à l'intérieur de la portion de paroi (2a) et comprend une surface contre laquelle l'air s'écoulant d'un côté amont entre en collision, la surface comprenant une portion intermédiaire faisant saillie vers le côté amont.

6. Climatiseur de véhicule (1) selon l'une quelconque des revendications 1 à 4, dans lequel l'élément de paroi définissant un espace (40) est disposé à l'extérieur de la portion de paroi (2a) du boîtier de climatisation (2).

7. Climatiseur de véhicule (1) selon l'une quelconque des revendications 1 à 5, dans lequel, dans les passages divisionnaires (3a, 3b), une portion de guidage (45, 40c) est disposée sur ou à proximité d'une portion de l'élément de paroi définissant un espace (40) où l'autre des portions de communication (402a, 402b) est formée, la portion de guidage (45, 40c) étant configurée pour favoriser l'introduction de l'air jusque dans l'autre des portions de communication (402a, 402b).

8. Climatiseur de véhicule (1) selon l'une quelconque des revendications 1 à 7, dans lequel l'espace d'ajout de fonction (41) défini par l'élément de paroi définissant un espace (40) comprend une portion intermédiaire ayant une superficie de section transversale de passage plus petite que les portions de communication.

9. Climatiseur de véhicule (1) selon l'une quelconque des revendications 1 à 8, dans lequel l'échangeur thermique (4, 5) comprend
un échangeur thermique de refroidissement (4) configuré pour refroidir l'air passant à travers celui-ci, et
un échangeur thermique de chauffage (5) disposé en aval de l'échangeur thermique de refroidissement (4) et configuré pour chauffer l'air passant à travers celui-ci, l'échangeur thermique de chauffage (5) étant disposé dans le passage d'air (3) de telle manière que des dérivations (301a, 301b, 302a, 302b) soient formées au-dessus et au-dessous de l'échangeur thermique de chauffage (5).

10. Climatiseur de véhicule (1) selon l'une quelconque des revendications 1 à 8, dans lequel l'échangeur thermique (4, 5) comprend
un échangeur thermique de refroidissement (4) configuré pour refroidir l'air passant à travers celui-ci, et
un échangeur thermique de chauffage (5) disposé en aval de l'échangeur thermique de refroidissement (4) et configuré pour chauffer l'air passant à travers celui-ci, l'échangeur thermique de chauffage (5) étant disposé dans le passage d'air (3) de telle manière que des dérivations (301a, 301b, 301a, 301b) soient formées seulement au-dessus et au-dessous de l'échangeur thermique de chauffage (5).

11. Climatiseur de véhicule (1) selon l'une quelconque des revendications 1 à 10, dans lequel la fonction ajoutée à l'air par le dispositif d'ajout de fonction (43) comprend au moins une fonction parmi une fonction d'ajout d'ions, une fonction de parfum, une fonction désodorisante, une fonction de régulation d'humidité, et une fonction de stérilisation.
